# EUROPEAN PATENT SPECIFICATION

(11) **EP 1 234 043 B1**
(45) Date of publication and mention of the grant of the patent: **10.03.2004**
(21) Application number: 00979809.1
(22) Date of filing: 04.12.2000
(51) Int. Cl.: C12N 15/62, C07K 14/33, C07K 19/00, A61K 47/48

(54) **CONSTRUCTS FOR DELIVERY OF THERAPEUTIC AGENTS TO NEURONAL CELLS**
KONSTRUKTE ZUR VERABREICHUNG VON THERAPEUTISCHEN WIRKSTOFFEN AN DIE NEURONZELLEN
CONSTRUCTIONS DESTINEES A LA DIFFUSION D'AGENTS THERAPEUTIQUES DANS DES CELLULES NEURONALES

(30) Priority: 02.12.1999 GB 9928530; 07.04.2000 GB 0008658
(43) Date of publication of application: 28.08.2002
(73) Proprietor: Health Protection Agency, Salisbury, Wiltshire SP4 0JG (GB)
(72) Inventor: Shone, Clifford Charles, Salisbury, Wiltshire SP4 0JG (GB); Sutton, John Mark, Salisbury, Wiltshire SP4 0JG (GB); Silman, Nigel, Salisbury, Wiltshire SP4 0JG (GB)
(74) Representative: Schlich, George William
(86) International application number: PCT/GB2000/004644
(87) International publication number: WO 2001/058936

(56) References cited:
- EP-A- 0 439 954
- WO-A-00/28041
- WO-A-01/36588
- WO-A-99/09057
- WO-A-99/17806

## Description

The present invention relates to constructs for delivering therapeutic substances to neuronal cells, to manufacture and use thereof, and in particular to constructs based on clostridial neurotoxins.

There are presently few effective treatments for major disorders of the central nervous system. Such disorders include neurodegenerative diseases, stroke, epilepsy, brain tumours, infections and HIV encephalopathy, and sufferers of these diseases far outnumber the morbidity of cancer and heart disease. The number of sufferers for CNS disorders such as stroke and the neurodegenerative diseases is set to grow, particularly in developed countries where the average age of the population is increasing. As our understanding of brain pharmacology increases and the underlying pathologies of diseases are elucidated, potential therapeutic strategies become apparent. All these treatments, however, face the formidable problem of efficient delivery of therapeutics to the various neuronal cell populations involved. Vectors which can effect efficient delivery to neuronal cells are thus required for a broad range of therapeutic substances, including drugs, enzymes, growth factors, therapeutic peptides and genes.

Ischemia/reperfusion injury induced by stroke or injury is one notable example in which rapid and efficient delivery of therapeutic agents would afford considerable benefit. Neurons injured by trauma or ischemia produce elevated levels of free oxygen radicals and release large amount of glutamate. These substances in high concentration are toxic to both neurons and surrounding cells which potentiate and amplify the damage process. Agents such as superoxide dismutase or glutamine synthetase which reduce the levels of these toxic substances have been shown to reduce neuronal cell death in a variety of *in vitro* and *in* vivo ischemia models (Gorovits *et al*. PNAS (1997) 94, 7024-7029; Francis *et al*. Experimental Neurology (1997) 146, 435-443; Lim *et al*. Ann. Thorac. Surg. (1986) 42, 282-286; Cuevas *et al*. Acta Anat. (1990) 137, 303-310). A major problem in the use of such therapies is the delivery of useful concentrations of the active agent to the site of trauma. Specific neuronal vectors could therefore play an important role in targeting such compounds to neuronal cells.

Peripheral nervous system disorders, such as motor neuron disease, are further examples of diseases which would benefit from the targeted delivery of therapeutic agents. Such therapies could take the form of drug delivery or DNA delivery via gene therapy strategies.

Gene therapy holds considerable promise for the treatment of neurodegenerative diseases such as Parkinson's and Alzheimer's diseases. Most of the currently available viral and non-viral gene delivery vectors lack tissue specificity which reduces both their efficiency and safety of use. Suitable neuronal cell-specific targeting ligands are therefore required for a broad range of gene vectors to enable effective treatments for neuronal diseases to be developed.

The botulinum neurotoxins are a family of protein toxins whose primary site of action is the neuromuscular junction where they block the release of the transmitter acetylcholine. The action of these toxins on the peripheral nervous system of man and animals results in the syndrome botulism, which is characterised by widespread flaccid muscular paralysis (Shone (1986) in 'Natural Toxicants in Foods', Editor D. Watson, Ellis Harwood, UK). Each of the botulinum neurotoxins consists of two disulphide-linked subunits; a 100 kDa heavy subunit which plays a role in the initial binding and internalisation of the neurotoxin into the nerve ending (Dolly et. al. (1984) Nature, 307, 457-460) and a 50 kDa light subunit which acts intracellularly to block the exocytosis process (McInnes and Dolly (1990) Febs Lett., 261, 323-326; de Paiva and Dolly (1990) Febs Lett., 277, 171-174).

The clostridial neurotoxins are potent inhibitors of calcium-dependent neurotransmitter secretion in neuronal cells. They are currently considered to mediate this activity through a specific endoproteolytic cleavage of at least one of three vesicle or pre-synaptic membrane associated proteins VAMP, syntaxin or SNAP-25 which are central to the vesicle docking and membrane fusion events of neurotransmitter secretion. The neuronal cell targeting of tetanus and botulinum neurotoxins is considered to be a receptor mediated event following which the toxins become internalised and subsequently traffic to the appropriate intracellular compartment where they effect their endopeptidase activity.

Clostridial neurotoxins share a common architecture of a catalytic L-chain (LC, ca 50 kDa) disulphide linked to a receptor binding and translocating H-chain (HC, ca 100 kDa). The HC polypeptide is considered to comprise all or part of two distinct functional domains. The carboxy-terminal half of the HC, termed the H_{C} domain (ca 50 kDa), is involved in the high affinity, neurospecific binding of the neurotoxin to cell surface receptors on the target neuron, whilst the amino-terminal half, termed the H_{N} domain (ca 50 kDa), is considered to mediate the translocation of at least some portion of the neurotoxin across cellular membranes such that the functional activity of the LC is expressed within the target cell. The H_{N} domain also has the property, under conditions of low pH, of forming ion-permeable channels in lipid membranes, and this may in some manner relate to its translocation function. For botulinum neurotoxin type A (BoNT/A) these domains are considered to reside within amino acid residues 872-1296 for the H_{C}, amino acid residues 449-871 for the H_{N} and residues 1-448 for the LC.

It is therefore possible to provide functional definitions of the domains within the neurotoxin molecule, as follows:-
(A) clostridial neurotoxin light chain:-
   - a metalloprotease exhibiting high substrate specificity for vesicle and/or plasma membrane associated proteins involved in the exocytotic process. In particular, it cleaves one or more of SNAP-25, VAMP (synaptobrevin / cellubrevin) and syntaxin.
(B) clostridial neurotoxin heavy chain H_{N} domain:-
   - a portion of the heavy chain which enables translocation of that portion of the neurotoxin molecule such that a functional expression of light chain activity occurs within a target cell.
   - the domain responsible for translocation of the endopeptidase activity, following binding of neurotoxin to its specific cell surface receptor via the binding domain, into the target cell.
   - the domain responsible for formation of ion-permeable pores in lipid membranes under conditions of low pH.
(c) clostridial neurotoxin heavy chain H_{C} domain:-
   - a portion of the heavy chain which is responsible for binding of the native holotoxin to cell surface receptor(s) involved in the intoxicating action of clostridial toxin prior to internalisation of the toxin into the cell.

The identity of the cellular recognition markers for these toxins is currently not understood and no specific receptor species have yet been identified although Kozaki et al. have reported that synaptotagmin may be the receptor for botulinum neurotoxin type B. It is probable that each of the neurotoxins has a different receptor.

Tetanus toxin is structurally very similar to botulinum neurotoxins but its primary site of action is the central nervous system where it blocks the release of inhibitory neurotransmitters from central synapses (Renshaw cells).

Tetanus and the botulinum neurotoxins from most of the seven serotypes, together with their derived heavy chains, have been shown to bind a wide variety of neuronal cell types with high affinities in the nM range, e.g. botulinum type B neurotoxin (Evans *et al*. (1986) Eur. J. Biochem. 154, 409-416).

However, a major obstacle to the use of the native clostridial heavy chain fragments as delivery vectors is that their highly aggregated state in solution prevent their adequate diffusion into body tissue and hence reduces their efficiency as targeting vectors. A further significant problem with any proposed clinical use of native tetanus toxin fragments as neuronal targeting ligands for therapeutics is the existence of circulating antibodies to the toxin in the majority of the population who have been immunized against tetanus. The presence of these antibodies is likely to reduce the efficacy of constructs based on tetanus toxin fragments. Thus, clostridial neurotoxin fragments do not offer solutions to the problems identified.

The present invention is based upon the discovery of the practical difficulties in using clostridial neurotoxin-based therapeutic compositions, and the devising of modified polypeptides and hybrid polypeptides based on clostridial neurotoxin fragments that avoid the aforementioned drawbacks.

EP-A-439 954 discloses a ternary hybrid protein, where there is a binding domain, at translocation domain and a chemical entity to be delivered into the cells. This document does not refer to the use of clostridial proteins as binding domains.

WO-A-99/09057 describes a method for *in vivo* delivery of a desired composition into the human or animal nervous system. The composition comprises a non-toxic, proteolytic fragment of tetanus toxin capable of binding nerve cells and being retrogradely transported through a synapse, in association with at least one molecule having a biological function.

WO-A-99/17806 describes a class of agents comprising a galactose-binding lectin linked to a derivative of a clostridial neurotoxin (L-chain). These agents may be used in the treatment of chronic pain.

Accordingly, a first aspect of the invention provides a composition comprising a therapeutic agent linked to a polypeptide, wherein the polypeptide is a non-toxic polypeptide, for delivery of a therapeutic agent to a neuronal cell, comprising:-
a binding domain that binds to the neuronal cell and is comprised of or derived
from clostridial heavy chain fragments and
a translocation domain that translocates the therapeutic agent into the neuronal cell,
wherein the translocation domain is not a H_{N} domain of a clostridial toxin and is not a fragment or derivative of a H_{N} domain of a clostridial toxin and wherein the therapeutic agent is for reduction of neuronal damage after ischemia/reperfusion or is for promotion of neuronal growth after damage.

In a further aspect, the invention also provides a composition comprising a therapeutic agent linked to a polypeptide, wherein the polypeptide is for delivery of a therapeutic agent to a neuronal cell, comprising:-
a binding domain that binds to the neuronal cell and is comprised of or derived from clostridial heavy chain fragments and
a translocation domain that translocates the therapeutic agent into the neuronal cell,
wherein the translocation domain is a non-aggregating translocation domain as measured by its size in physiological buffer and wherein the therapeutic agent is for reduction of neuronal damage after ischemia/reperfusion or is for promotion of neuronal growth after damage.

Whether the construct is an aggregating one is usually apparent from a lack of solubility of the construct, and this may be seen upon simple visual inspection of the construct in aqueous media: non-aggregating domains result in constructs of the invention that are partially or preferably totally soluble whereas aggregating domains result in non-soluble aggregates of polypeptides having apparent sizes of many tens or even hundreds the size of a single polypeptide. Generally, the construct should be non-aggregating as measured by size on gel electrophoresis, and the size or apparent size of the construct measured should preferably be less than 5.0 x 10⁵ daltons, more preferably less than 1.5 x 10⁵ daltons, with the measuring being suitably carried out on native PAGE using physiological conditions.

A still further aspect of the invention provides a composition comprising a therapeutic agent linked to a polypeptide, wherein the polypeptide is for delivery of a therapeutic agent to a neuronal cell, comprising:-
a binding domain that binds to the neuronal cell and is comprised of or derived from clostridial heavy chain fragments, and
a translocation domain that translocates the therapeutic agent into the neuronal cell,
wherein the translocation domain is selected from (1) a H_{N} domain of a diphtheria toxin, (2) a fragment or derivative of (1) that substantially retains the translocating activity of the H_{N} domain of a diphtheria toxin, (3) a fusogenic peptide, (4) a membrane disrupting peptide, and (5) translocating fragments and derivatives of (3) and (4) and wherein the therapeutic agent is for reduction of neuronal damage after ischemia/reperfusion or is for promotion of neuronal growth after damage.

It is to be noted that botulinum toxin C₂ is not a neurotoxin as it has no neuronal specificity, instead it is an enterotoxin and suitable for use in the invention to provide a non-aggregating translocation domain.

A yet further aspect of the invention provides a composition comprising a therapeutic agent linked to a polypeptide, wherein the polypeptide is for delivery of a therapeutic agent to a neuronal cell, comprising:-
a binding domain that binds to the neuronal cell and is comprised of or derived from clostridial heavy chain fragments, and
a translocation domain that translocates the therapeutic agent into the neuronal cell,
wherein the polypeptide has reduced affinity to neutralising antibodies to tetanus toxin compared with the affinity to such antibodies of native tetanus toxin and wherein the therapeutic agent is for reduction of neuronal damage after ischemia/reperfusion or is for promotion of neuronal growth after damage.

The above aspects may singly or in any combination be exhibited by polypeptides of the invention and thus a typical preferred polypeptide of the invention (i) lacks the neurotoxic activities of botulinum and tetanus toxins, (ii) displays high affinity to neuronal cells corresponding to the affinity of a clostridial neurotoxin for those cells, (iii) contains a domain which can effect translocation across cell membranes, and (iv) occurs in a less aggregated state than the corresponding heavy chain from botulinum or tetanus toxin in physiological buffers.

A significant advantage of the polypeptides of the invention is their non-aggregated state, thus rendering them usable as soluble polypeptides where the prior art constructs were not and overcoming most if not all of the drawbacks of previous constructs based upon clostridial neurotoxins.

The polypeptides according to the invention generally include sequences from the H_{C} domains of the botulinum and tetanus neurotoxins and these are combined with functional domains from other proteins, such that the essential functions of the native heavy chain, binding to neuronal cells, is retained. Thus, for example, the H_{C} domain of botulinum type F neurotoxin is fused to the translocation domain derived from diphtheria toxin to give a modified clostridial heavy chain fragment. Surprisingly, such polypeptides are more useful as constructs for delivering substances to neuronal cells than are the native clostridial heavy chains.

Thus, according to a preferred aspect of the invention there is provided a polypeptide having an amino acid sequence comprising (a) a sub-sequence based on the H_{C} fragment of botulinum or tetanus neurotoxin, and (b) a sub-sequence based on a translocation domain, e.g. from diphtheria toxin, that is not derived from a clostridial neurotoxin, and wherein the said polypeptide (i) lacks the neurotoxin activities of botulinum and tetanus toxins; (ii) displays high affinity to neuronal cells, (iii) contains a domain which can effect translocation across cell membranes and (iv) occurs in a less aggregated state than the corresponding heavy chain of botulinum or tetanus toxin in physiological buffers.

The modified clostridial heavy chain is suitably produced by combining the binding domain (H_{C} domain) of a clostridial neurotoxin with a non-clostridial translocation domain. Thus, for example, a modified clostridial heavy chain fragment may be constructed from the translocation domain of diphtheria toxin (residues 194-386) fused to the H_{C} domain of a botulinum toxin (e.g. type F H_{C} fragment, residues 865-1278; type A H_{C} fragment, residues 872-1296).

In another embodiment of the invention, the modified clostridial heavy chain is produced by combining the H_{C} domain of a clostridial neurotoxin with a membrane disrupting peptide which functions as a translocation domain, suitably a viral peptide. Thus, for example, a modified clostridial heavy chain fragment may be constructed by combining the H_{C} domain of a botulinum toxin with a peptide based on influenza virus haemagglutinin HA2 (residues 1-23).

The polypeptides of the invention have properties which make them useful as neuronal targeting ligands; they are non-toxic and yet retain the specific, high affinity binding to neuronal cells displayed by the botulinum or tetanus toxins. Unlike the native clostridial heavy chains, however, the modified clostridial heavy chains occur in a less aggregated state in solution which improves their access to neuronal cells. The preferred constructs are soluble in aqueous solution, in contrast to the highly aggregated state of the prior art constructs.

In another aspect of the invention, there is provided a modified tetanus heavy chain fragment which, in addition to the properties of modified heavy chains defined above, has the added advantage in that it has reduced affinity to neutralizing antibodies, present as a result of anti-tetanus inoculation, compared to the native tetanus toxin heavy chain. The polypeptides according to this aspect of the invention generally include subsequences derived from the heavy chain of tetanus toxin (residues 458 - 1315) and from which epitopes responsible for the immunogenicity of tetanus toxin have optionally been reduced or removed. Thus, for example, it is desirable to eliminate immunogenic epitopes associated with H_{C} domain as well as that of the H_{N} domain. Although it is possible to eliminate epitopes by deleting small numbers of amino acids (e.g. less than 20 or preferably less than 10 amino acids), it has been found that epitopes associated with immunogenicity of tetanus toxin heavy chain can be reduced more rigorously by replacing a large number of amino acid residues (e.g. at least 100, at least 200 and preferably 400 or more residues) with amino acid sequences from other toxins.

Thus according to a preferred aspect of the invention related to modified tetanus heavy chains, there is provided a polypeptide having an amino sequence comprising (a) an H_{N} domain derived from a non-clostridial source (e.g. diphtheria toxin), (b) one or more subsequences derived from the sequence of a botulinum H_{C}, and (c) one or more subsequences derived from the sequence of tetanus toxin H_{C}, and wherein said polypeptide (i) lacks the neurotoxin activities of botulinum and tetanus toxins, (ii) displays high affinity to neuronal cells corresponding to the neuronal binding of tetanus neurotoxin, (iii) contains a domain which can effect translocation across cell membranes and (iv) has low affinity to neutralizing antibodies to tetanus toxin which are present as result of anti-tetanus inoculation.

This latter modified tetanus heavy chain fragment can be produced by combining the binding domain (H_{C} domain) of tetanus neurotoxin with a non-clostridial translocation domain. Thus, for example, a modified tetanus heavy chain fragment may be constructed from the translocation domain of diphtheria toxin (residues 1 94-386) fused to the H_{C} domain of a tetanus toxin (residues 865-1315).

In another embodiment of the invention the modified tetanus heavy chain is derived a non-clostridial translocation domain fused to the H_{C} domain of a botulinum toxin into which the minimal domains of tetanus toxin are inserted to confer tetanus toxin-like binding activity onto the resulting hybrid. Thus, for example a modified tetanus heavy chain may be constructed from the translocation domain of diphtheria toxin (residues 194-386) fused to the H_{C} domain of a botulinum type F fragment (residues 865-1278) in which residues 1097-1273 of the latter have been replaced by homologous sequences from tetanus toxin.

The modified tetanus heavy chains have properties which make them useful as neuronal targeting ligands; they are non-toxic and yet retain the specific, high affinity binding to neuronal cells displayed by tetanus toxin. Unlike native tetanus toxin binding fragments, however, the modified clostridial binding fragments have different immunogenic properties which makes them more useful clinically. Specifically, the different immunogenic properties of the modified clostridial binding fragments of the invention significantly reduce the problems caused by existing antibodies to native tetanus toxin sequences.

While the use of modified heavy chains based on botulinum neurotoxins as neuronal targeting ligands does not suffer from the problem of pre-existing circulating antibodies, tetanus toxin is unique amongst the clostridial toxins in that it has selectivity to inhibitory neurons (e.g. Renshaw cells) and as such the modified tetanus toxin heavy chains are valuable targeting ligands for this class of neuron. Tetanus toxin also has the property that it can retrograde transport from the peripheral to the central nervous system.

In another embodiment of the invention, the modified clostridial heavy chain fragment is fused to a linker peptide via the N-terminus of the translocation domain to which a polypeptide payload may be attached. An examples of such a linker peptide is the sequence CGLVPAGSGP (SEQ ID NO:1) which contains the thrombin protease cleavage site and a cysteine residue for disulphide bridge formation. Such a peptide linker allows production of a recombinant fusion protein comprising a polypeptide therapeutic molecule fused by the linker peptide to the N-terminus of the modified clostridial heavy chain fragment. The latter single chain fusion protein may then be treated with thrombin to give a dichain protein in which the polypeptide therapeutic is linked to the translocation domain of the modified clostridial heavy chain fragment by a disulphide link. In another example of a linker peptide in which the translocation domain does not contain a free cysteine residue near its C-terminus, such as is the case when the translocation domain is a fusogenic peptide, the linker peptide contains both cysteine residues required for the disulphide bridge. An example of the latter linker peptide is the amino acid sequence: CGLVPAGSGPSAGSSAC (SEQ ID NO:2).

In another embodiment of the invention, the modified clostridial heavy chain is linked to a polypeptide which may be an enzyme, growth factor, protein or peptide which has therapeutic benefits when delivered to neuronal cells. The polypeptide may be linked to the modified clostridial heavy chain by chemical means. Alternatively the polypeptide may be produced as a fusion protein linked to the modified clostridial binding fragment by recombinant technology using the linker peptides as described above. In such an example, the construct would contain the following components:-
a polypeptide therapeutic substance;
a linker peptide; and
a modified clostridial heavy chain

An example of a polypeptide therapeutic payload is superoxide dismutase.

In yet another embodiment of the invention, the modified clostridial heavy chain is linked directly or indirectly to DNA such that the construct is capable of delivering the DNA to neuronal cells, e.g. via the receptor for tetanus toxin. Such constructs have gene therapy applications and be used to switch on, or off, selected genes with the cell. The DNA may be contained within a liposome or be condensed via a peptide or protein. The modified clostridial heavy chain may be chemically linked to the protein that effects the DNA condensation by chemical coupling agents. Alternatively, the modified clostridial heavy chain may be produced as a fusion protein, by recombinant technology, with a peptide that can effect the condensation of DNA.

In yet another embodiment of the invention, the modified clostridial heavy chain fragment may be linked to a recombinant virus such that the modified virus has an altered tropism and is capable of transducing cells via the tetanus toxin receptor. Such a construct is of use to correct genetic defects within neuronal cells by switching on, or off, selected genes. The modified clostridial heavy chain fragment may be linked directly to the surface of the virus using chemical cross-linking agents. Alternatively the modified clostridial heavy chain fragment may be linked to the recombinant virus via an antibody which specifically bind to the virus. In this instance the modified clostridial binding fragment is chemically coupled to a polyclonal or monoclonal antibody which specifically recognizes a marker on the surface of the virus. A similar modified clostridial binding fragment-antibody fusion protein could be produced by recombinant technology in which the antibody component is a recombinant single chain antibody.

In yet another embodiment of the invention, the modified clostridial heavy chain fragment is linked to a drug release system such as a microparticle constructed from a suitable polymer, e.g. poly (lactide-co-glycolide), polyhydroxylalkonate, collagen, poly(divinyl-ether-comaleic anhydride, poly (styrene-co-maleic anhydride) or other polymer useful in such microparticles. The modified clostridial heavy chain fragment may be linked to the drug release system by covalent chemical coupling, or electrostatic or hydrophobic forces. The modified clostridial heavy chain fragment may also be encapsulated within the release vehicle together with the therapeutic payload provided that a portion of the modified clostridial binding fragment is exposed at the surface. Alternatively, the modified clostridial heavy chain fragment may be linked, at either the N- or C-terminal end, to a peptide or protein to facilitate coupling of the fragment to the drug release system.

Other strategies are known by which modified heavy chain binding fragments can be linked to range of therapeutic substances using a variety of established chemical cross-linking techniques, and a variety of fusion proteins can be produced containing a modified clostridial binding fragment and another polypeptide. Using these techniques a variety of substances can be targeted to neuronal cells using the modified clostridial binding fragments. Examples of possible uses of the modified clostridial binding fragments as neuronal delivery vectors are given in more detail below in Table 1.

Constructs of the invention may be introduced into either neuronal or non-neuronal tissue using methods known in the art. By subsequent specific binding to neuronal cell tissue, the targeted construct exerts its therapeutic effects. Ideally, the construct is injected near a site requiring therapeutic intervention.

The construct of the invention may be produced as a suspension, emulsion, solution or as a freeze dried powder depending on the application and properties of the therapeutic substance. The construct of the invention may be resuspended or diluted in a variety of pharmaceutically acceptable liquids depending on the application.

"Clostridial neurotoxin" means either tetanus neurotoxin or one of the seven botulinum neurotoxins, the latter being designated as serotypes A, B C₁, D, E, F or G.

"Modified clostridial heavy chain fragment" means a polypeptide fragment which binds to neuronal cell receptors in similar manner to a corresponding heavy chain derived from botulinum or tetanus toxins but differs in its amino acid sequence and properties compared to the corresponding fragment derived from tetanus toxin.

"Bind" in relation to the botulinum and tetanus heavy chain fragments, means the specific interaction between the clostridial fragment and one or more cell surface receptors or markers which results in localization of the binding fragment on the cell surface. In the case of the clostridial neurotoxins, the property of a fragment being able to 'bind' like a fragment of a given serotype can be demonstrated by competition between the ligand and the native toxin for its neuronal cell receptor.

"High affinity binding specific to neuronal cell corresponding to that of a clostridial neurotoxin" refers to the ability of a ligand to bind strongly to cell surface receptors of neuronal cells that are involved in specific binding of a given neurotoxin. The capacity of a given ligand to bind strongly to these cell surface receptors may be assessed using conventional competitive binding assays. In such assays radiolabelled clostridial neurotoxin is contacted with neuronal cells in the presence of various concentrations of non-radiolabelled ligands. The ligand mixture is incubated with the cells, at low temperature (0-3°C) to prevent ligand internalization, during which competition between the radiolabelled clostridial neurotoxin and non-labelled ligand may occur. In such assays when the unlabelled ligand used is the same as that of the labelled neurotoxin, the radiolabelled clostridial neurotoxin will be displaced from the neuronal cell receptors as the concentration of non-labelled neurotoxin is increased. The competition curve obtained in this case will therefore be representative of the behaviour of a ligand which shows "high affinity binding specificity to neuronal cells corresponding to that of a clostridial neurotoxin", as used herein.

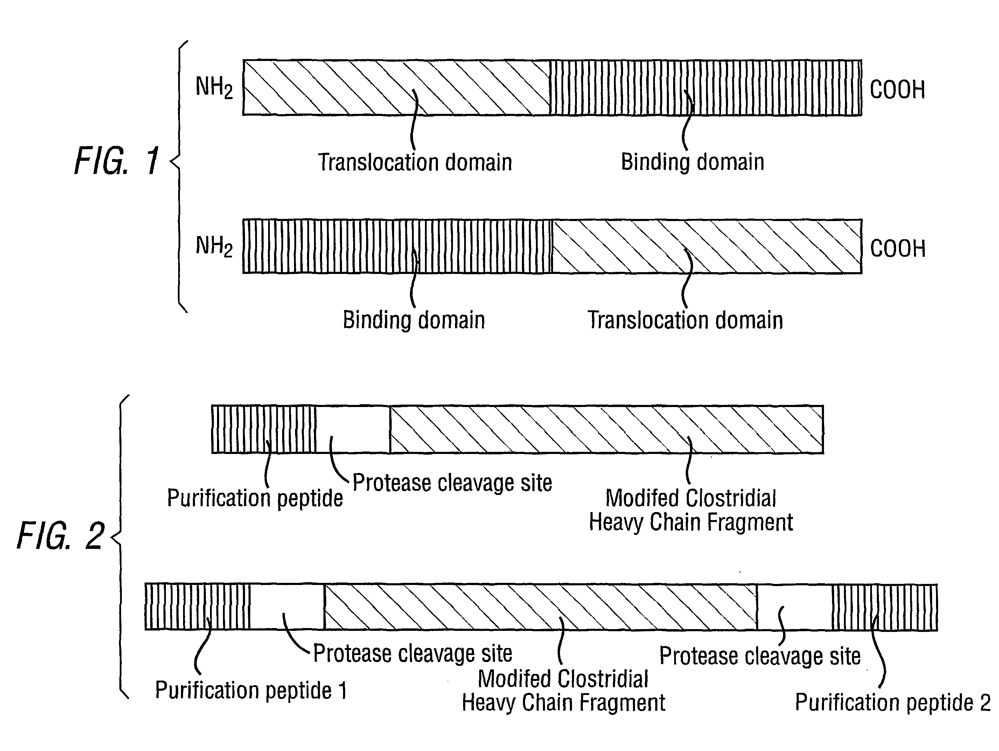

"Translocation domain" means a domain or fragment of a protein which effects transport of itself and/or other proteins and substances across a membrane or lipid bilayer. The latter membrane may be that of an endosome where translocation will occur during the process of receptor-mediated endocytosis. Translocation domains can frequently be identified by the property of being able to form measurable pores in lipid membranes at low pH (Shone *et al*. Eur J. Biochem. 167, 175-180). Examples of translocation domains are set out in more detail below in Figure 1. in the application, translocation domains are frequently referred to as "H_{N} domains".

"Translocation" in relation to translocation domain, means the internalization events which occur after binding to the cell surface. These events lead to the transport of substances into the cytosol of neuronal cells.

"Therapeutic substances" or "agents" mean any substance, agent or mixture thereof, which, if delivered by the modified clostridial binding fragment, would be beneficial to the treatment of neuronal diseases. Examples of these include drugs, growth factors, enzymes, and DNA packaged in various forms (e.g. modified viruses, cationic liposomes, and condensed DNA).

Also provided in the present invention are methods of manufacture of the polypeptides of the invention by expressing in a host cell a nucleic acid encoding the polypeptide, and the use of a polypeptide or a composition according to the invention in the treatment of a disease state associated with neuronal cells.

The invention is now illustrated in the following specific embodiments and accompanied by drawings in which:-
Fig. 1 shows modified clostridial heavy chain fragments produced by recombinant technology as a fusion proteins;
Fig. 2 shows modified clostridial heavy chain fragments produced by recombinant technology; fusion proteins may contain one or more purification peptide tags to assist in the purification of the protein; one or more protease cleavage sites may also be included to enable removal of the purification peptide tags; similar purification strategies may also be employed for modified clostridial binding fragments containing a translocation domain;

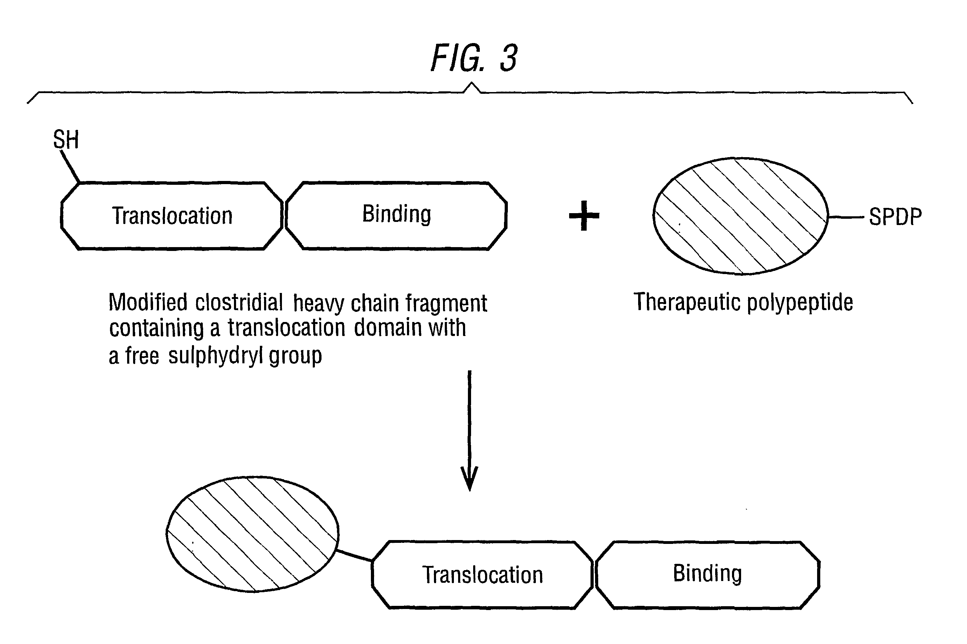

Fig. 3 shows linkage of a modified clostridial binding fragment to a therapeutic substance; the modified clostridial heavy chain contains a translocation domain which has a free thiol group (an example of translocation domain with this property is amino acid sequence 194-386 of diphtheria toxin), a free amino group on the therapeutic substance is modified with a cross-linking reagent (e.g. SPDP; Pierce & Warriner, UK Ltd.) which will subsequently allow conjugate formation using the free thiol present on the modified clostridial binding fragment;

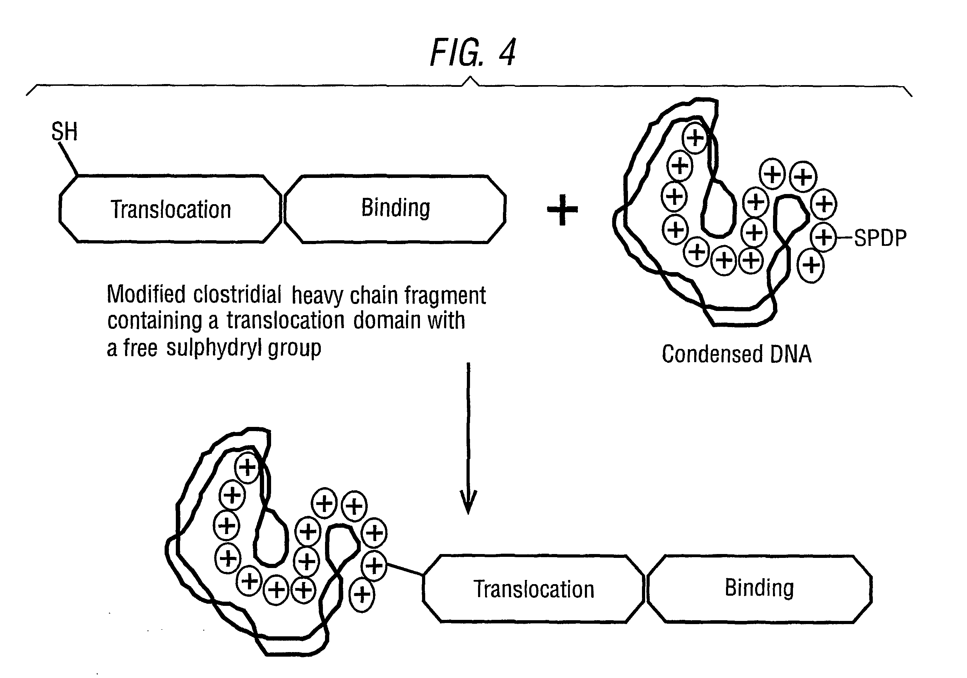

Fig. 4 shows the formation of a conjugate between a modified clostridial heavy chain fragment and an oligonucleotide as described in Example 4;

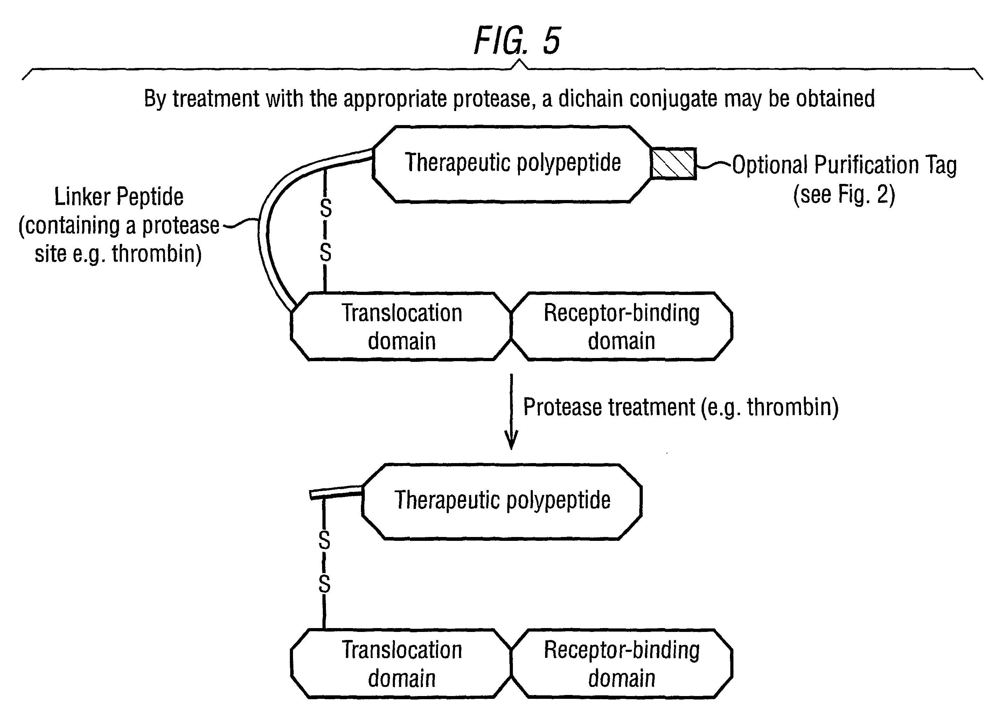

Fig. 5 shows a strategy for producing a recombinant modified clostridial heavy chain as a fusion protein with a polypeptide therapeutic substance. The latter is fused to the modified clostridial heavy chain by a linker peptide. The linker peptide contains a unique protease cleavage site (e.g. that recognized by thrombin) and a cysteine residue. Examples of linker peptides are (a) CGLVPAGSGP; and (b) CGIEGRAPGP (SEQ ID NO:18). The cysteine residue forms a disulphide bridge with an another available cysteine residue on the translocation domain of the modified heavy chain fragment. If desirable, then by treatment with thrombin, a dichain product may be produced in which the polypeptide therapeutic substance is linked to the heavy chain via a disulphide bridge;

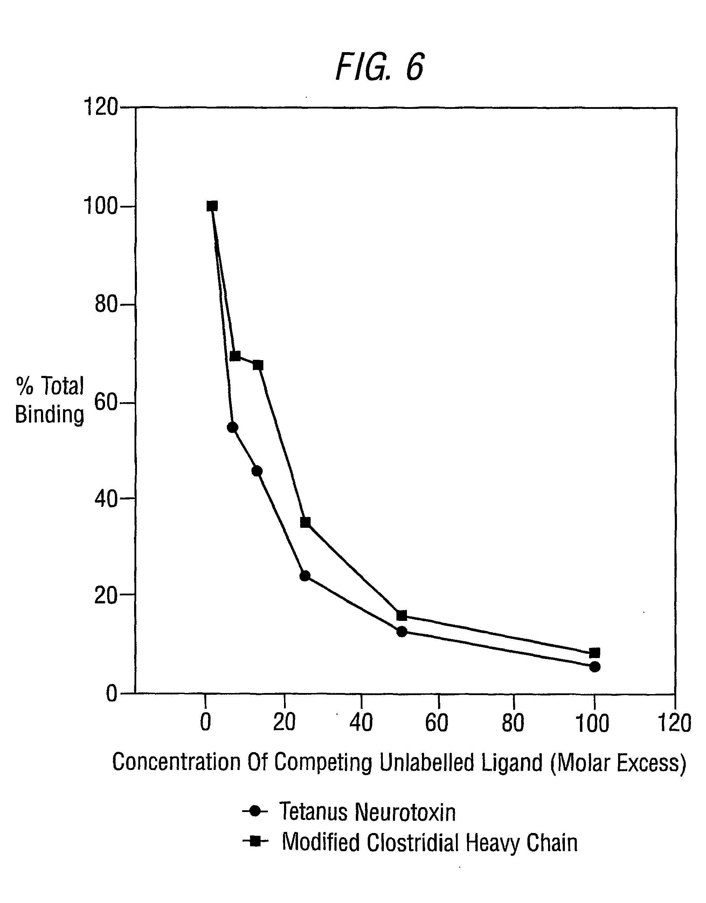

Fig. 6 shows a comparison of the binding of a modified heavy chain with that of the native neurotoxin to neuronal synaptic membranes, the modified heavy chain displaying the binding characteristics of tetanus neurotoxin as assessed by the method described in Example 7;

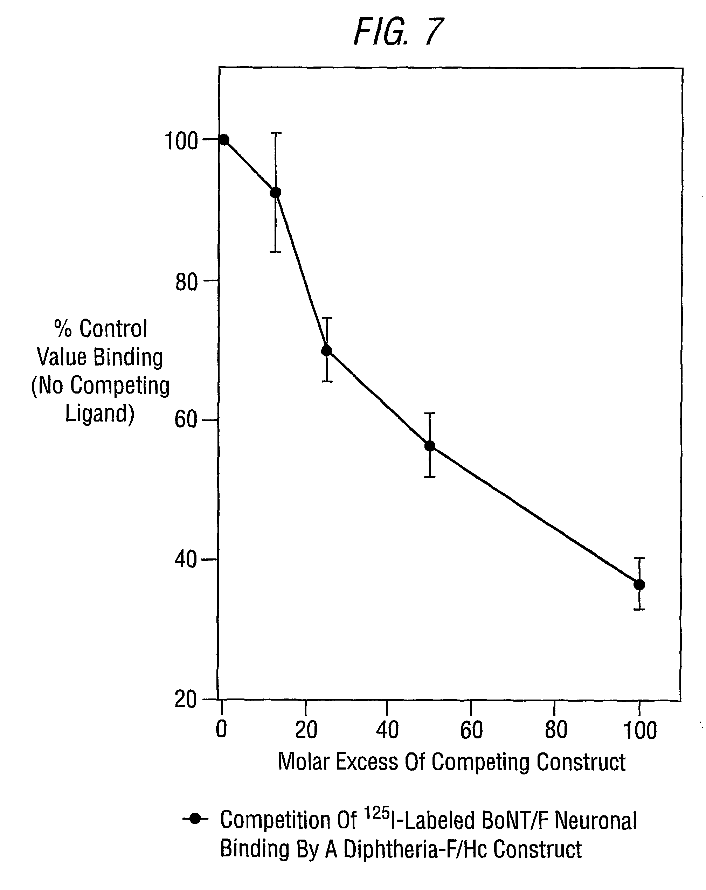

Fig. 7 shows the binding to neuronal membranes of a modified clostridial heavy chain based on the binding domain of botulinum type F neurotoxin; in this example, modified heavy chain contained the translocation (H_{N}) domain of diphtheria toxin and the binding (H_{c}) domain of type F neurotoxin; and

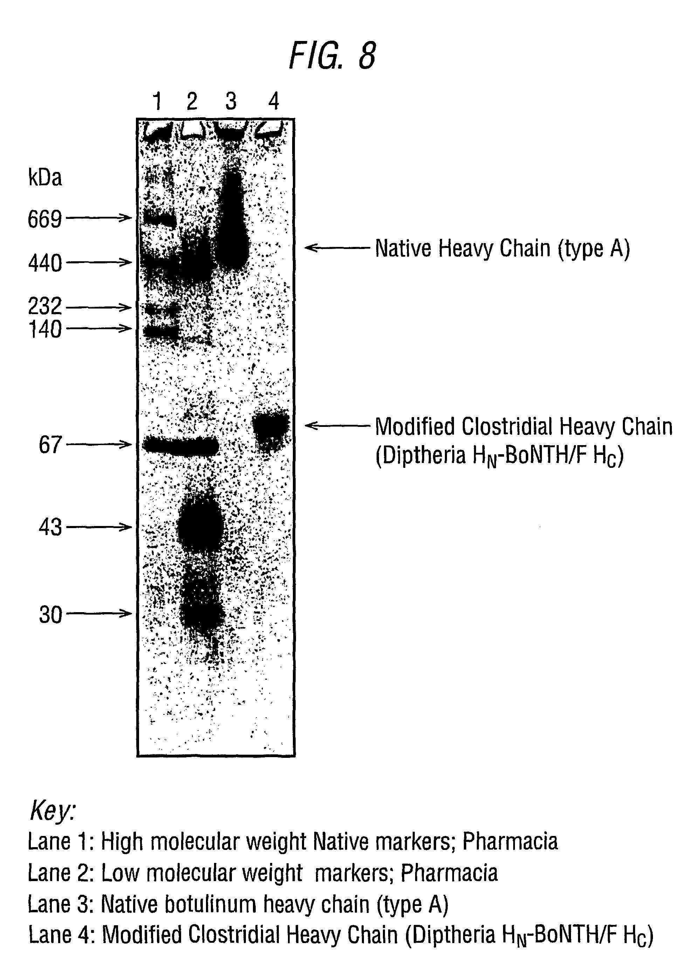

Fig. 8 shows a comparison of the molecular sizes, under non-denaturing conditions, of a modified clostridial heavy chain compared to a native heavy chain; the modified clostridial heavy chain (Diphtheria H_{N} - BoNT/F H_{c}) runs as a monomer of approximately 70kDa while a native heavy chain (from BoNT/A) runs as an aggregate of > 500kDa.

In more detail, figure 1 shows examples of embodiments of the invention incorporating modified clostridial heavy chain fragments.

The binding domain is derived from sequences of the clostridial neurotoxins:-
(a) H_{c} domains, e.g.
   BoNT/A residues 872-1296
   BoNT/B residues 859-1291
   BoNT/C residues 867-1291
   BoNT/D residues 863-1276
   BoNT/E residues 846-1252
   BoNT/F residues 865-1278
   BoNT/G residues 864-1297
   Tetanus residues 880-1315
(b) Hybrid H_{c} domains, e.g.
   hybrids of the H_{c} domain of BoNT/F and tetanus
(c) Truncated H_{c} domains

The translocation domain may be derived from a number of sources:-
(a) Bacterial toxins, e.g. diphtheria toxin fragment B (residues 194-386)
(b) Viral fusogenic peptides, e.g. from influenza virus haemagglutinin HA-2
(c) Synthetic membrane disrupting peptides (e.g. Plank *et al*., J. Biol. Chem., 269, 12918-12924).

Figure 2 shows examples of Recombinant Modified Clostridial Heavy Chain Fragment Fusion Proteins Showing Positions of Purification Peptide Tags and Specific Protease Cleavage Sites (by treatment with the appropriate protease, the purification peptide tags may be removed from the modified clostridial binding fragment).

Examples of purification peptides tags are:
His6
S peptide
T7 peptide
Calmodulin binding peptide
Maltose binding protein

Examples of specific protease cleavage sites are:-
Thrombin
Enterokinase
Factor X

### Example 1.

### Preparation and purification of a recombinant modified clostridial heavy chain fragments.

Standard molecular biology protocols were used for all genetic manipulations (*e.g.* Sambrook *et al*. 1989, Molecular Cloning a Laboratory Manual, Second Edition, Cold Spring Harbor Laboratory Press, Cold Spring Harbor, New York). An entirely synthetic gene encoding the H_{C} regions of botulinum toxin from *C. botulinum* type F (residues 865-1278) and tetanus toxin (residues 880-1315) were generated using Recursive PCR reactions (Prodromou & Pearl 1992, *Protein Engineering,* 5: 827-829) using self-priming oligonucleotides containing the desired sequence. The codon bias and GC/AT base ratio was adjusted for ease of expression in *E*. *coli*. Fragments were cloned sequentially into pLitmus 38 (New England Biolabs, Inc., Beverly, MA) to assemble the entire gene. Constructs for expression were sub-cloned into pMALc2 (NEB) replacing the *Bam*H1-*Eco*R1 fragment. The ligation reactions were transformed into *E.coli* JM109 (Promega).

Plasmid DNA was amplified, purified and screened for the presence of the appropriate sequence (Ausubel *et al*. 1989, Current Protocols in Molecular Biology, John Wiley & Sons, New York). Gene constructions confirmed as possessing the correct sequences were then transformed into the expression host *E. coli* BL21 (DE3) (Studier & Moffatt 1986, *Journal of Molecular Biology,* 189: 113-130).

Additional sequences for adding affinity purification tags and one or more specific protease site for the subsequent removal of these affinity tags were also included in the reading frame of the gene products.

The recombinant proteins expressed in pMAL were produced with amino-terminal maltose-binding protein tags allowing proteins to be purified by affinity chromatography on amylose resin. Briefly, cultures of *E.coli* BL21 (DE3) pMALc2-H_{C} were grown in Terrific broth-ampicillin (100 µgml⁻¹)-kanamycin (30 µgml⁻¹) to an OD₆₀₀ nm of 2.5-3.8, and protein expression was induced by the addition of 1 mM IPTG for approximately 2 h. Cells were lysed by freeze/thaw followed by sonication, lysates cleared by centrifugation and supernatants loaded onto an amylose resin column and eluted with maltose. All buffers used were as specified by the manufacturer. Thrombin or factor Xa protease sites were included within the protein for subsequent removal of these purification tags.

Other coding sequences which enable expression of the desired protein would also be acceptable. Other tags or linking sites may also be incorporated into the sequence. Examples of some of these options are summarized in Figure 2.

### Example 2.

### Production of a modified clostridial heavy chain fragments.

Using the techniques described in Example 1, modified clostridial heavy chain fragments was constructed by fusing domains of the H_{c} fragments of either botulinum type F or tetanus neurotoxins with the translocation domain of diphtheria toxin. The amino acid sequences of examples are shown in SEQ ID NO:s 8-17, which also gives examples of modified tetanus heavy chains in which the H_{c} fragment is a hybrid of tetanus and botulinum type F neurotoxin.

### Example 3.

Coupling of a modified clostridial heavy chain fragment to a protein or an enzyme.

The polypeptide, protein or enzyme to be linked to the modified clostridial heavy chain fragment is first derivatized with a suitable cross-linking agent. Mn-Superoxide dismutase (SOD) was modified by treatment with a 15 molar excess of SPDP (Pierce) in 0.05M Hepes buffer pH 7.0 containing 0.15M NaCl for 60 min at 25°C. The excess SPDP was removed by dialysis against the same buffer At 4°C for 16h. The substituted SOD was then mixed in a 1:5 molar ration with modified clostridial heavy chain fragment fused to a translocation domain derived from diphtheria toxin (see Figure 3) and incubated at 25°C for 16h. After incubation the SOD-modified clostridial binding fragment conjugate was purified by gel filtration chromatography on Sephadex G200.

### Example 4.

### Coupling of modified clostridial heavy chain fragment to condensed DNA.

Poly-L-lysine (Mᵣ 1000-4000) (10 mg) to be used for the condensation of DNA was dissolved in 2ml of 20mM Hepes buffer pH 7.4 containing 0.15M NaCl (HBS). To this solution 0.6mg of Sulpho-LC-SPDP (Pierce and Warriner, UK Ltd.) was added and the mixture incubated for 30 min at 25°C. The activated poly-L-lysine was then dialysed against HBS at 4°C using a dialysis tubing of 1000 molecular weight cut-off and then diluted to 1mg/ml using HBS.

Condensation of DNA was carried out in glass tubes. Purified plasmid DNA containing a gene encoding a therapeutic protein (or a reporter gene) under the control of a suitable promoter (e.g. CMV immediate early, or a neuronal-specific promoter e.g. neuron-specific enolase promoter) was made 1 mg/ml in HBS and added to glass tubes followed by the activated poly-L-lysine as prepared above. Activated poly-L-lysine is added in various proportions to the DNA (see Table 2) and incubated for 90min at 25°C.

**Table 2**

| **Condensation of DNA with activated poly-L-lysine.** | | | |
|---|---|---|---|
| Sample no. | DNA(µg) | Activated Poly-L-lysine | HBS |
| 1 | 750 | 250 | 1500 |
| 2 | 1500 | 500 | 500 |
| 3 | 500 | 250 | 1750 |
| 4 | 1000 | 500 | 1000 |

After incubation the size of the condensed DNA particles was assessed using a Brookhaven BI90 particle sizer. The incubation conditions giving the highest proportion of condensed DNA particle of less than 100nM in diameter was used to produce DNA-modified clostridial binding fragment conjugates. Modified clostridial heavy chain was dialysed against HBS.

The dialysed fragments (100µg) was then added to 1 ml of condensed DNA and incubated for 18h at 25°C to from the modified clostridial binding protein-condensed DNA construct (see Figure 4).

### Example 5.

### Delivery of DNA to a neuronal cells via the modified clostridial heavy chain fragment receptor.

Modified clostridial heavy chain-condensed DNA construct described in Example 4 was diluted with 2ml MEM serum free medium. Growth media from NG 108 grown in 12 well dished was removed and 1 ml of the diluted construct added and incubated for 2h at 37°C in the presence of 5% CO₂. Growth media (1ml) was then added to each well and the incubation continued under the same conditions for 24-48h. After this period the cell were examined.

In experiments were the condensed DNA contained a reporter gene encoding Green Fluorescent Protein, several of the cells showed visible expression of the reporter protein illustrating successful delivery of the DNA into the neuronal cell. Various control experiments were conducted to confirm the observed transfection in NG108 cells was receptor mediated:-
Transfection of NG108 cells was found to be dependent on the presence of modified clostridial heavy chain fragment within conjugates (no transfection was observed with condensed particles DNA alone)
No transfection was observed in non-neuronal cells (Vero cells) using the heavy chain-DNA conjugates.

### Example 6.

### Preparation of conjugates of modified clostridial heavy chain fragment and microparticles consisting of poly (lactide-co-glycolide).

398mg of poly (lactide co-glycolide) low internal viscosity (3000 MW)(Beohringer Mannheim, ) was dissolved in 4ml dichloromethane. This was homogenised at 2000rpm for 150 seconds with 1 ml of buffer solution containing the therapeutic substance, such as an enzymes and/or drugs. In the case of Mn superoxide dismutase, 10mg of the enzyme was dissolved in 10mM Hepes buffer pH 8.0 containing 100mM NaCl. The mixture was then added to 50ml of 8% poly vinyl alcohol and emulsified at 2000rpm for a further 1 50 seconds. The emulsion was poured into 300ml of ultrapure distilled water at 37°C and stirred for 30min at 37°C. The microparticles were collected by centrifugation at 10000 x g for 25min at 20°C and then resuspended in 300ml water and centrifuged as above. This washing procedure was the repeated a further 4 times. After the final centrifugation the water supernatant fluid was removed and the microparticles freeze dried.

2mg of poly (lactide-co-glycolide) microparticles were re-suspended in 1ml of activation buffer (01.M MES buffer, pH 6.0 containing 0.5M NaCl). Solid 1-Ethyl-3-[3-dimethylaminopropyl] carbodiimide (EDC) and N-hydroxysulphosuccinimide (sulphoNHS) were added to 2mM and 5mM respectively and the mixture incubated for 15 min at 25°C. The microparticles were washed by centrifugation for 1 min at 10000 x g and resuspension in 1ml of activation buffer. The wash step was repeated 4 times and then the microparticles resuspended in 1ml of activation buffer containing 33µM of a modified clostridial heavy chain fragment and incubated for 2 h at 25°C. The reaction was then quenched with 10mM hydroxylamine. After 20min at 25°C the microparticles were washed in a suitable buffer by centrifugation as described above.

### Example 7.

### Demonstration of the high affinity binding to neuronal cell tissue displayed by modified heavy chain fragments

Clostridial neurotoxins may be labelled with 125-iodine using chloramine-T and its binding to various cells assessed by standard methods such as described in Evans *et al*. 1986, Eur J. Biochem., 154, 409 or Wadsworth *et al*. 1990, Biochem. J. 268, 123). In these experiments the ability of modified clostridial heavy chain constructs to compete with native clostridial neurotoxins for receptors present on neuronal cells or brain synaptosomes was assessed. All binding experiments were carried out in binding buffers. For the botulinum neurotoxins this buffer consisted of: 50mM hepes pH 7.0, 30mM NaCl, 0.25% sucrose, 0.25% bovine serum albumin. For tetanus toxin, the binding buffer was: 0.05M MES buffer pH 6.0 containing 0.6% bovine serum albumin. In a typical binding experiment the radiolabelled clostridial neurotoxin was held at a fixed concentration of between 1-10nM. Reaction mixtures were prepared by mixing the radiolabelled toxin with various concentrations of unlabelled neurotoxin or modified clostridial heavy chain construct. The reaction mixture were then added to neuronal cells or rat brain synaptosomes and then incubated at 0-3°C for 2hr. After this period the neuronal cells of synaptosomes were washed twice with binding ice-cold binding buffer and the amount of labelled clostridial neurotoxin bound to cells or synaptosomes was assessed by γ-counting.

In an experiment using a modified clostridial heavy construct which consisted of a binding domain derived from tetanus toxin and a translocation domain from diphtheria toxin, the construct was found to compete with ¹²⁵I-labelled tetanus neurotoxin for neuronal cell receptors in a similar manner to unlabelled native tetanus neurotoxin (see Figure 6). These data showed that the construct had retained binding properties of the native neurotoxin.

In a further experiment using Diphtheria H_{N} - BoNT/F H_{c} as the modified clostridial heavy chain, the construct was found to compete with ¹²⁵I-labelled BoNT/F for receptors on neuronal synaptic membranes (Figure 7). These data indicate that the modified clostridial heavy chain retains the neuronal receptor-binding properties of BoNT/F.

### Example 8.

**Non-denaturing gel electrophoresis to compare the sizes of a native botulinum toxin heavy chain (type A) with that of a modified clostridial heavy chain (recombinant Diphtheria H**_{**N**} **- BoNT/F H**_{**C**}**)**

Botulinum type A heavy chain was purified as described previously (Shone *et al*. 1985 Eur J. Biochemistry 151, 75-82) and recombinant Diphtheria H_{N} - BoNT/F H_{C} purified as described in Examples1 and 2. The modified clostridial heavy chain was purifies as a Maltose Binding Protein fusion with then the fusion protein removed by treatment with Factor Xa. Samples of type A heavy chain (20µg) and Diphtheria H_{N} - BoNT/F H_{C} (10µg) were loaded on a 4-20% Tris-glycine polyacrylamide gel in Tris-glycine buffer. Samples were electrophoresed to equilibrium (Novex gel system; 43 volts 16 hours) and the gel stained with Coomassie blue. The results are shown in Figure 8. The major band for Diphtheria H_{N} - BoNT/F /H_{C} appears to migrate very close to its predicted molecular weight of approx 70kDa. In contrast, the native type A heavy chain appears as a diffuse band at approximately 500kDa, compared to an estimated molecular weight of 100kDa, which suggesting the formation of large protein aggregates.

### Example 9.

### Recombinant modified heavy chain-superoxide dismutase conjugates.

Recombinant modified heavy chain-superoxide dismutase conjugates were prepared comprising a combination of the following elements:-
- a bacterial superoxide dismutase, from *Bacillus stearothermophilus*;
- a linker region which allows the formation of a disulphide bond between the superoxide dismutase and the translocation domain and which also contains a unique protease cleavage site for cleavage by factor Xa or thrombin to allow the formation of a dichain molecule;
- a translocation domain from diphtheria toxin or a endosomolytic (fusogenic) peptide from influenza virus haemagglutinin); and
- a neuronal cell-specific binding domain from tetanus or botulinum neurotoxin type F.

The sequences of these recombinant modified heavy chain-superoxide dismutase conjugates are shown in SEQ ID NO:s 3-7.

To confirm the nature of their structure, the recombinant modified clostridial heavy chain-superoxide dismutase conjugates were converted to the dichain form by treatment with a unique protease corresponding to the cleavage site sequences within the linker region. Conjugates containing the thrombin cleavage site were treated with thrombin (20µg per mg of conjugate) for 20h at 37°C; conjugates containing the factor Xa cleavage site were treated with factor Xa (20µg per mg of conjugate) for 20 min at 22°C.

On SDS-PAGE gels, under non-reducing conditions, the conjugates appeared as a band of molecular mass approx. 120kDa. In the presence of reducing agent (dithiothreitol) two bands were observed at approx. molecular masses 70 and 30kDa corresponding to the modified clostridial heavy chain and superoxide dismutase respectively. These data illustrate that, after treatment with the unique protease, the conjugates consist of the latter two components which are linked by a disulphide bridge.

**TABLE 1.**

| Examples of Potential Therapeutic Uses of Modified Clostridial Binding Fragments | | |
|---|---|---|
| **Therapeutic Substance or Effector** | **Site and Mechanism of Action** | **Potential Clinical Effects** |
| (a) Enzymes:- | | |
| | | |
| Superoxide dismutase | Reduce oxidative stress after stroke/injury of brain or spinal cord | Reduction of neuronal damage after ischemia/ reperfusion |
| | | |
| Glutamine synthetase | Reduce damage by excess glutamate after stroke/injury of the brain or spinal cord | Reduction of neuronal damage after ischemia/ reperfusion |
| | | |
| (b) Antibodies:- | | |
| | | |
| Anti-tetanus toxin | Neutralize the action of tetanus toxin at the spinal cord | Reverse the effects of intoxication by tetanus toxin |
| | | |
| Anti SNARE protein (e.g. SNAP-25, VAMPs Syntaxins) | Modulate neurotransmitter release | Hyper secretory disorders |
| | | |
| © Viruses/DNA | | |
| | | |
| Viral gene therapy vectors (e.g. adenovirus, herpes simplex, etc.) | Replacement of defective genes within the CNS | Treatment of neurodegenerative diseases (Parkinson's' Alzheimer's ALS etc.) and other neuronal diseases |
| | | |
| Non-viral vectors for gene therapy (e.g. liposomes) | Replacement of defective genes within the CNS | Treatment of neurodegenerative diseases and other neuronal diseases |
| | | |
| (d) Growth factors | | |
| | | |
| e.g. BDNF, CTNF, NGF | Deliver growth factors to the brain and spinal cord | Treatment of neurodegenerative diseases, promotion of neuronal growth after damage. |
| | | |
| (e) Anti-viral agents | Deliver anti-viral agents to the brain or spinal cord neurons | Treatment of latent viral infections within neuronal cells, e.g. HIV, herpes simplex infections |
| | | |
| (f) Anti-cancer agents | Deliver cytotoxic agents to neoplastic cells of the CNS | Treatment of neuronal neoplasia |

### SEQUENCES OF MODIFIED CLOSTRIDIAL HEAVY CHAIN - SUPEROXIDE DISMUTASE CONJUGATES

### SEQ ID NO: 3

Construct containing:
MnSOD from *B.stearothermophilus*
a linker that can be cleaved by thrombin
a translocation domain derived from diphtheria toxin
a binding domain from tetanus toxin

MPFELPALPYPYDALEPHIDKETMNIHHTKHHNTYVTNLNAALEGHPDLQNKSLEELL
SNLEALPESIRTAVRNNGGGHANHSLFWTILSPNGGGEPTGELADAINKKFGSFTAFK
DEFSKAAAGRFGSGWAWLVVNNGELEITSTPNQDSPIMEGKTPILGLDVWEHAYYL
KYQNRRPEYIAAFWNVVNWDEVAKRYSEAKPKSGSCGLVPRGSGPGSSVGSSLSCI
NLDWDVIRDKTKTKIESLKEHGPIKNKMSESPNKTVSEEKAKQYLEEFHQTALEHPELS
ELKTVTGTNPVFAGANYAAWAVNVAQVIDSETADNLEKTTAALSILPGIGSVMGIAD
GAVHHNTEEIVAQSIALSSLMVAQAIPLVGELVDIGFAAYNFVESIINLFQVVHNSYN
RSAYSPGHKTQPFLHDGYAVSWNTVRSKNLDCWVDNEEDIDVILKKSTILNLDINNDI
ISDISGFNSSVITYPDAQLVPGINGKAIHLVNNESSEVIVHKAMDIEYNDMFNNFTVSF
WLRVPKVSASHLEQYGTNEYSIISSMKKHSLSIGSGWSVSLKGNNLIWTLKDSAGEV
RQITFRDLPDKFNAYLANKWVFITITNDRLSSANLYINGVLMGSAEITGLGAIREDNNIT
LKLDRCNNNNQYVSIDKFRIFCKALNPKEIEKLYTSYLSITFLRDFWGNPLRYDTEYYLI
PVASSSKDVQLKNITDYMYLTNAPSYTNGKLNIYYRRLYNGLKFIIKRYTPNNEIDSFV
KSGDFIKLYVSYNNNEHIVGYPKDGNAFNNLDRILRVGYNAPGIPLYKKMEAVKLRDL
KTYSVQLKLYDDKNASLGLVGTHNGQIGNDPNRDILIASNWYFNHLKDKILGCDWYF
VPTDEGWTNDLQ

### SEQ ID NO: 4

Construct containing:
MnSOD from *B.stearothermophilus*
a linker that can be cleaved by factor Xa
a translocation domain derived from diphtheria toxin
a binding domain from botulinum type F toxin

MPFELPALPYPYDALEPHIDKETMNIHHTKHHNTYVTNLNAALEGHPDLQNKSLEELL
SNLEALPESIRTAVRNNGGGHANHSLFWTILSPNGGGEPTGELADAINKKFGSFTAFK
DEFSKAAAGRFGSGWAWLVVNNGELEITSTPNQDSPIMEGKTPILGLDVWEHAYYL
KYQNRRPEYIAAFWNVVNWDEVAKRYSEAKPKSGSCGIEGRAPGPGSSVGSSLSCI
NLDWDVIRDKTKTKIESLKEHGPIKNKMSESPNKTVSEEKAKQYLEEFHQTALEHPELS
ELKTVTGTNPVFAGANYAAWAVNVAQVIDSETADNLEKTTAALSILPGIGSVMGIAD
GAVHHNTEEIVAQSIALSSLMVAQAIPLVGELVDIGFAAYNFVESIINLFQVVHNSYN
RSAYSPGHKTQPFLHDGYAVSWNTVRSTMSYTNDKILILYFNKLYKKIKDNSILDMRY
ENNKFIDISGYGSNISINGDVYIYSTNRNQFGIYSSKPSEVNIAQNNDIIYNGRYQNFSIS
FWVRIPKYFNKVNLNNEYTIIDCIRNNNSGWKISLNYNKIIWTLQDTAGNNQKLVFNY
TQMISISDYINKWIFVTITNNRLGNSRIYINGNLIDEKSISNLGDIHVSDNILFKIVGCNDT
RYVGIRYFKVFDTELGKTEIETLYSDEPDPSILKDFWGNYLLYNKRYYLLNLLRTDKSIT
QNSNFLNINQQRGVYQKPNIFSNTRLYTGVEVIIRKNGSTDISNTDNFVRKNDLAYINV
VDRDVEYRLYADISIAKPEKIIKLIRTSNSNNSLGQIIVMDSIGNNCTMNFQNNNGGNI
GLLGFHSNNLVASSWYYNNIRKNTSSNGCFWSFISKEHGWQEN

### SEQ ID NO: 5

Construct containing:
a mitochondrial leader sequence from human MnSOD
MnSOD from *B.stearothermophilus*
a linker that can be cleaved by factor Xa
a translocation domain derived from diphtheria toxin
a binding domain from tetanus toxin

MLSRAVCGTSRQLAPALGYLGSRQKHSRGSPALPYPYDALEPHIDKETMNIHHTKHH
NTYVTNLNAALEGHPDLQNKSLEELLSNLEALPESIRTAVRNNGGGHANHSLFWTILS
PNGGGEPTGELADAINKKFGSFTAFKDEFSKAAAGRFGSGWAWLVVNNGELEITSTP
NQDSPIMEGKTPILGLDVWEHAYYLKYQNRRPEYIAAFWNVVNWDEVAKRYSEAKP
KSGSCGIEGRAPGPGSSVGSSLSCINLDWDVIRDKTKTKIESLKEHGPIKNKMSESPN
KTVSEEKAKQYLEEFHQTALEHPELSELKTVTGTNPVFAGANYAAWAVNVAQVIDS
ETADNLEKTTAALSILPGIGSVMGIADGAVHHNTEEIVAQSIALSSLMVAQAIPLVGEL
VDIGFAAYNFVESIINLFQVVHNSYNRSAYSPGHKTQPFLHDGYAVSWNTVRSKNLD
CWVDNEEDIDVILKKSTILNLDINNDIISDISGFNSSVITYPDAQLVPGINGKAIHLVNNE
SSEVIVHKAMDIEYNDMFNNFTVSFWLRVPKVSASHLEQYGTNEYSIISSMKKHSLSI
GSGWSVSLKGNNLIWTLKDSAGEVRQITFRDLPDKFNAYLANKWVFITITNDRLSSA
NLYINGVLMGSAEITGLGAIREDNNITLKLDRCNNNNQYVSIDKFRIFCKALNPKEIEKL
YTSYLSITFLRDFWGNPLRYDTEYYLIPVASSSKDVQLKNITDYMYLTNAPSYTNGKL
NIYYRRLYNGLKFIIKRYTPNNEIDSFVKSGDFIKLYVSYNNNEHIVGYPKDGNAFNNLD
RILRVGYNAPGIPLYKKMEAVKLRDLKTYSVQLKLYDDKNASLGLVGTHNGQIGNDP
NRDILIASNWYFNHLKDKILGCDWYFVPTDEGWTNDLQ

### SEQ ID NO: 6

Construct containing:
a mitochondrial leader sequence from human MnSOD
MnSOD from *B.stearothermophilus*
a linker that can be cleaved by thrombin
a translocation domain derived from diphtheria toxin
a binding domain from botulinum type F toxin

MLSRAVCGTSRQLAPALGYLGSRQKHSRGSPALPYPYDALEPHIDKETMNIHHTKHH
NTYVTNLNAALEGHPDLQNKSLEELLSNLEALPESIRTAVRNNGGGHANHSLFWTILS
PNGGGEPTGELADAINKKFGSFTAFKDEFSKAAAGRFGSGWAWLVVNNGELEITSTP
NQDSPIMEGKTPILGLDVWEHAYYLKYQNRRPEYIAAFWNVVNWDEVAKRYSEAKP
KSGSCGLVPRGSGPGSSVGSSLSCINLDWDVIRDKTKTKIESLKEHGPIKNKMSESPN
KTVSEEKAKQYLEEFHQTALEHPELSELKTVTGTNPVFAGANYAAWAVNVAQVIDS
ETADNLEKTTAALSILPGIGSVMGIADGAVHHNTEEIVAQSIALSSLMVAQAIPLVGEL
VDIGFAAYNFVESIINLFQVVHNSYNRSAYSPGHKTQPFLHDGYAVSWNTVRSTMS
YTNDKILILYFNKLYKKIKDNSILDMRYENNKFIDISGYGSNISINGDVYIYSTNRNQFGI
YSSKPSEVNIAQNNDIIYNGRYQNFSISFWVRIPKYFNKVNLNNEYTIIDCIRNNNSGW
KISLNYNKIIWTLQDTAGNNQKLVFNYTQMISISDYINKWIFVTITNNRLGNSRIYINGN
LIDEKSISNLGDIHVSDNILFKIVGCNDTRYVGIRYFKVFDTELGKTEIETLYSDEPDPSIL
KDFWGNYLLYNKRYYLLNLLRTDKSITQNSNFLNINQQRGVYQKPNIFSNTRLYTGVE
VIIRKNGSTDISNTDNFVRKNDLAYINVVDRDVEYRLYADISIAKPEKIIKLIRTSNSNNS
LGQIIVMDSIGNNCTMNFQNNNGGNIGLLGFHSNNLVASSWYYNNIRKNTSSNGCF
WSFISKEHGWQEN

### SEQ ID NO: 7

Construct containing:
MnSOD from *B.stearothermophilus*
a linker that can be cleaved by factor Xa
a translocation peptide from influenza virus
a binding domain from botulinum type F toxin

MPFELPALPYPYDALEPHIDKETMNIHHTKHHNTYVTNLNAALEGHPDLQNKSLEELL
SNLEALPESIRTAVRNNGGGHANHSLFWTILSPNGGGEPTGELADAINKKFGSFTAFK
DEFSKAAAGRFGSGWAWLVVNNGELEITSTPNQDSPIMEGKTPILGLDVWEHAYYL
KYQNRRPEYIAAFWNVVNWDEVAKRYSEAKPKSGSCGIEGRAPGPGSSVGSSLSCI
NGLFGAIAGFIENGWEGMIDGWYGTMSYTNDKILILYFNKLYKKIKDNSILDMRYENN
KFIDISGYGSNISINGDVYIYSTNRNQFGIYSSKPSEVNIAQNNDIIYNGRYQNFSISFW
VRIPKYFNKVNLNNEYTIIDCIRNNNSGWKISLNYNKIIWTLQDTAGNNQKLVFNYTQ
MISISDYINKWIFVTITNNRLGNSRIYINGNLIDEKSISNLGDIHVSDNILFKIVGCNDTRY
VGIRYFKVFDTELGKTEIETLYSDEPDPSILKDFWGNYLLYNKRYYLLNLLRTDKSITQN
SNFLNINQQRGVYQKPNIFSNTRLYTGVEVIIRKNGSTDISNTDNFVRKNDLAYINVVD
RDVEYRLYADISIAKPEKIIKLIRTSNSNNSLGQIIVMDSIGNNCTMNFQNNNGGNIGLL
GFHSNNLVASSWYYNNIRKNTSSNGCFWSFISKEHGWQEN

### Protein sequence for Diphtheria toxin translocation domain with BoNT/F-H_{C}

SEQ ID NO: 8

GSSVGSSLSCINLDWDVIRDKTKTKIESLKEHGPIKNKMSESPNKTVSEEKAKQYLEEF
HQTALEHPELSELKTVTGTNPVFAGANYAAWAVNVAQVIDSETADNLEKTTAALSIL
PGIGSVMGIADGAVHHNTEEIVAQSIALSSLMVAQAIPLVGELVDIGFAAYNFVESIIN
LFQVVHNSYNRPAYSPGHKTQPFLHDGYAVSWNTVRSTMSYTNDKILILYFNKLYKK
IKDNSILDMRYENNKFIDISGYGSNISINGDVYIYSTNRNQFGIYSSKPSEVNIAQNNDII
YNGRYQNFSISFWVRIPKYFNKVNLNNEYTIIDCIRNNNSGWKISLNYNKIIWTLQDTA
GNNQKLVFNYTQMISISDYINKWIFVTITNNRLGNSRIYINGNLIDEKSISNLGDIHVSD
NILFKIVGCNDTRYVGIRYFKVFDTELGKTEIETLYSDEPDPSILKDFWGNYLLYNKRYY
LLNLLRTDKSITQNSNFLNINQQRGVYQKPNIFSNTRLYTGVEVIIRKNGSTDISNTDNF
VRKNDLAYINVVDRDVEYRLYADISIAKPEKIIKLIRTSNSNNSLGQIIVMDSIGNNCTM
NFQNNNGGNIGLLGFHSNNLVASSWYYNNIRKNTSSNGCFWSFISKEHGWQEN

### Protein sequence for Diphtheria toxin translocation domain with TeNT-H_{C}

SEQ ID NO: 9

GSSVGSSLSCINLDWDVIRDKTKTKIESLKEHGPIKNKMSESPNKTVSEEKAKQYLEEF
HQTALEHPELSELKTVTGTNPVFAGANYAAWAVNVAQVIDSETADNLEKTTAALSIL
PGIGSVMGIADGAVHHNTEEIVAQSIALSSLMVAQAIPLVGELVDIGFAAYNFVESIIN
LFQVVHNSYNRPAYSPGHKTQPFLHDGYAVSWNTVRSKNLDCWVDNEEDIDVILKK
STILNLDINNDIISDISGFNSSVITYPDAQLVPGINGKAIHLVNNESSEVIVHKAMDIEYN
DMFNNFTVSFWLRVPKVSASHLEQYGTNEYSIISSMKKHSLSIGSGWSVSLKGNNLI
WTLKDSAGEVRQITFRDLPDKFNAYLANKWVFITITNDRLSSANLYINGVLMGSAEIT
GLGAIREDNNITLKLDRCNNNNQYVSIDKFRIFCKALNPKEIEKLYTSYLSITFLRDFWG
NPLRYDTEYYLIPVASSSKDVQLKNITDYMYLTNAPSYTNGKLNIYYRRLYNGLKFIIK
RYTPNNEIDSFVKSGDFIKLYVSYNNNEHIVGYPKDGNAFNNLDRILRVGYNAPGIPLY
KKMEAVKLRDLKTYSVQLKLYDDKNASLGLVGTHNGQIGNDPNRDILIASNWYFNHL
KDKILGCDWYFVPTDEGWTNDLQ

### Protein sequence for Diphtheria toxin translocation domain TeNT-H_{C} domain II

SEQ ID NO: 10

GSSVGSSLSCINLDWDVIRDKTKTKIESLKEHGPIKNKMSESPNKTVSEEKAKQYLEEF
HQTALEHPELSELKTVTGTNPVFAGANYAAWAVNVAQVIDSETADNLEKTTAALSIL
PGIGSVMGIADGAVHHNTEEIVAQSIALSSLMVAQAIPLVGELVDIGFAAYNFVESIIN
LFQVVHNSYNRPAYSPGHKTQPFLHDGYAVSWNTVRSVYVSIDKFRIFCKALNPKEI
EKLYTSYLSITFLRDFWGNPLRYDTEYYLIPVASSSKDVQLKNITDYMYLTNAPSYTN
GKLNIYYRRLYNGLKFIIKRYTPNNEIDSFVKSGDFIKLYVSYNNNEHIVGYPKDGNAFN
NLDRILRVGYNAPGIPLYKKMEAVKLRDLKTYSVQLKLYDDKNASLGLVGTHNGQIG
NDPNRDILIASNWYFNHLKDKILGCDWYFVPTDEGWTNDLQ

### Protein sequence for Diphtheria toxin translocation domain with truncated TeNT-H_{C}

SEQ ID NO: 11

GSSVGSSLSCINLDWDVIRDKTKTKIESLKEHGPIKNKMSESPNKTVSEEKAKQYLEEF
HQTALEHPELSELKTVTGTNPVFAGANYAAWAVNVAQVIDSETADNLEKTTAALSIL
PGIGSVMGIADGAVHHNTEEIVAQSIALSSLMVAQAIPLVGELVDIGFAAYNFVESIIN
LFQVVHNSYNRPAYSPGHKTQPFLHDGYAVSWNTVRSVYNNESSEVIVHKAMDIEY
NDMFNNFTVSFWLRVPKVSASHLEQYGTNEYSIISSMKKHSLSIGSGWSVSLKGNNL
IWTLKDSAGEVRQITFRDLPDKFNAYLANKWVFITITNDRLSSANLYINGVLMGSAEIT
GLGAIREDNNITLKLDRCNNNNQYVSIDKFRIFCKALNPKEIEKLYTSYLSITFLRDFWG
NPLRYDTEYYLIPVASSSKDVQLKNITDYMYLTNAPSYTNGKLNIYYRRLYNGLKFIIK
RYTPNNEIDSFVKSGDFIKLYVSYNNNEHIVGYPKDGNAFNNLDRILRVGYNAPGIPLY
KKMEAVKLRDLKTYSVQLKLYDDKNASLGLVGTHNGQIGNDPNRDILIASNWYFNHL
KDKILGCDWYFVPTDEGWTNDLQ

### Protein sequence for Diphtheria toxin translocation domain BoNT/F-H_{C} domain I TeNT-H_{C} domain II

SEQ ID NO: 12

GSSVGSSLSCINLDWDVIRDKTKTKIESLKEHGPIKNKMSESPNKTVSEEKAKQYLEEF
HQTALEHPELSELKTVTGTNPVFAGANYAAWAVNVAQVIDSETADNLEKTTAALSIL
PGIGSVMGIADGAVHHNTEEIVAQSIALSSLMVAQAIPLVGELVDIGFAAYNFVESIIN
LFQVVHNSYNRPAYSPGHKTQPFLHDGYAVSWNTVRSTMSYTNDKILILYFNKLYKK
IKDNSILDMRYENNKFIDISGYGSNISINGDVYIYSTNRNQFGIYSSKPSEVNIAQNNDII
YNGRYQNFSISFWVRIPKYFNKVNLNNEYTIIDCIRNNNSGWKISLNYNKIIWTLQDTA
GNNQKLVFNYTQMISISDYINKWIFVTITNNRLGNSRIYINGNLIDEKSISNLGDIHVSD
NILFKIVGCNDTRYVSIDKFRIFCKALNPKEIEKLYTSYLSITFLRDFWGNPLRYDTEYYLI
PVASSSKDVQLKNITDYMYLTNAPSYTNGKLNIYYRRLYNGLKFIIKRYTPNNEIDSFV
KSGDFIKLYVSYNNNEHIVGYPKDGNAFNNLDRILRVGYNAPGIPLYKKMEAVKLRDL
KTYSVQLKLYDDKNASLGLVGTHNGQIGNDPNRDILIASNWYFNHLKDKILGCDWYF
VPTDEGWTNDLQ

### Protein sequence for Diphtheria toxin translocation domain

SEQ ID NO: 13

SVGSSLSCINLDWDVIRDKTKTKIESLKEHGPIKNKMSESPNKTVSEEKAKQYLEEFH
QTALEHPELSELKTVTGTNPVFAGANYAAWAVNVAQVIDSETADNLEKTTAALSILP
GIGSVMGIADGAVHHNTEEIVAQSIALSSLMVAQAIPLVGELVDIGFAAYNFVESIINL
FQVVHNSYNRPAYSPGHKTQPFLHDGYAVSWNTVRS

### Protein sequence for Clostridium botulinum C2 enterotoxin translocation domain with BoNT/F-H_{C}

SEQ ID NO: 14

(a)

LVSKFENSVKNSNKNYFTINGLMGYYFENDFFNLNIISPTLDGNLTFSKEDINSILGNKII
KSARWIGLIKPSITGEYILSTNSPNCRVELNGEIFNLSLNTSNTVNLIQGNVYDIRIEQLM
SENQLLKNYEGIKLYWETSDIIKEIIPSEVLLKPNYSNTNEKSKFIPNNTLFSNAKLKANA
NRDTDRDGIPDEWEINGYTVMNQKAVAWDDKFAANGYKKYVSNPFKPCTANDPYT
DFEKVSGQIDPSVSMVARDPMISAYPIVGVQMERLVVSKSETITGDSTKSMSKSTSH
SSTNINTVGAEVSGSLQLAGGIFPVFSMSASANYSHTWQNTSTVDDTTGESFSQGL
SINTGESAYINPNIRYYNTGTAPVYNVTPTTTIVIDKQSVATIKGQESLIGDYLNPGGT
YPIIGEPPMALNTMDQFSSRLIPINYNQLKSIDNGGTVMLSTSQFTGNFAKYNSNGNL
VTDGNNWGPYLGTIKSTTASLTLSFSGQTTQVAVVAPNFSDPEDKTPKLTLEQALVK
AFALEKKNGKFYFHGLEISKNEKIQVFLDSNTNNDFENQLKNTADKDIMHCIIKRNMNI
LVKVITFKENISSINIINDTNFGVQSMTGLSNRSKGQDGIYRAATTAFSFKSKELKYPE
GRYRMRFVIQSYEPFTTMSYTNDKILILYFNKLYKKIKDNSILDMRYENNKFIDISGYGS
NISINGDVYIYSTNRNQFGIYSSKPSEVNIAQNNDIIYNGRYQNFSISFWVRIPKYFNKV
NLNNEYTIIDCIRNNNSGWKISLNYNKIIWTLQDTAGNNQKLVFNYTQMISISDYINKW
IFVTITNNRLGNSRIYINGNLIDEKSISNLGDIHVSDNILFKIVGCNDTRYVGIRYFKVFDT
ELGKTEIETLYSDEPDPSILKDFWGNYLLYNKRYYLLNLLRTDKSITQNSNFLNINQQRG
VYQKPNIFSNTRLYTGVEVIIRKNGSTDISNTDNFVRKNDLAYINVVDRDVEYRLYADI
SIAKPEKIIKLIRTSNSNNSLGQIIVMDSIGNNCTMNFQNNNGGNIGLLGFHSNNLVAS
SWYYNNIRKNTSSNGCFWSFISKEHGWQEN

SEQ ID NO: 15

(b)

LVSKFENSVKNSNKNYFTINGLMGYYFENDFFNLNIISPTLDGNLTFSKEDINSILGNKII
KSARWIGLIKPSITGEYILSTNSPNCRVELNGEIFNLSLNTSNTVNLIQGNVYDIRIEQLM
SENQLLKNYEGIKLYWETSDIIKEIIPSEVLLKPNYSNTNEKSKFIPNNTLFSNAKLKANA
NRDTDRDGIPDEWEINGYTVMNQKAVAWDDKFAANGYKKYVSNPFKPCTANDPYT
DFEKVSGQIDPSVSMVARDPMISAYPIVGVQMERLVVSKSETITGDSTKSMSKSTSH
SSTNINTVGAEVSGSLQLAGGIFPVFSMSASANYSHTWQNTSTVDDTTGESFSQGL
SINTGESAYINPNIRYYNTGTAPVYNVTPTTTIVIDKQSVATIKGQESLIGDYLNPGGT
YPIIGEPPMALNTMDQFSSRLIPINYNQLKSIDNGGTVMLSTSQFTGNFAKYNSNGNL
VTDGNNWGPYLGTIKSTTASLTLSFSGQTTQVAVVAPNFSDPEDKTPKLTLEQALVK
AFALEKKNGKFYFHGLEISKNEKIQVFLDSNTNNDFENQLKNTADKDIMHCIIKRNMNI
LVKVITFKENISSINTMSYTNDKILILYFNKLYKKIKDNSILDMRYENNKFIDISGYGSNIS
INGDVYIYSTNRNQFGIYSSKPSEVNIAQNNDIIYNGRYQNFSISFWVRIPKYFNKVNL
NNEYTIIDCIRNNNSGWKISLNYNKIIWTLQDTAGNNQKLVFNYTQMISISDYINKWIF
VTITNNRLGNSRIYINGNLIDEKSISNLGDIHVSDNILFKIVGCNDTRYVGIRYFKVFDTE
LGKTEIETLYSDEPDPSILKDFWGNYLLYNKRYYLLNLLRTDKSITQNSNFLNINQQRG
VYQKPNIFSNTRLYTGVEVIIRKNGSTDISNTDNFVRKNDLAYINVVDRDVEYRLYADI
SIAKPEKIIKLIRTSNSNNSLGQIIVMDSIGNNCTMNFQNNNGGNIGLLGFHSNNLVAS
SWYYNNIRKNTSSNGCFWSFISKEHGWQEN

### Protein sequence for Clostridium botulinum C2 enterotoxin translocation domain with Tetanus-H_{C}

SEQ ID NO: 16

(a)

LVSKFENSVKNSNKNYFTINGLMGYYFENDFFNLNIISPTLDGNLTFSKEDINSILGNKII
KSARWIGLIKPSITGEYILSTNSPNCRVELNGEIFNLSLNTSNTVNLIQGNVYDIRIEQLM
SENQLLKNYEGIKLYWETSDIIKEIIPSEVLLKPNYSNTNEKSKFIPNNTLFSNAKLKANA
NRDTDRDGIPDEWEINGYTVMNQKAVAWDDKFAANGYKKYVSNPFKPCTANDPYT
DFEKVSGQIDPSVSMVARDPMISAYPIVGVQMERLVVSKSETITGDSTKSMSKSTSH
SSTNINTVGAEVSGSLQLAGGIFPVFSMSASANYSHTWQNTSTVDDTTGESFSQGL
SINTGESAYINPNIRYYNTGTAPVYNVTPTTTIVIDKQSVATIKGQESLIGDYLNPGGT
YPIIGEPPMALNTMDQFSSRLIPINYNQLKSIDNGGTVMLSTSQFTGNFAKYNSNGNL
VTDGNNWGPYLGTIKSTTASLTLSFSGQTTQVAVVAPNFSDPEDKTPKLTLEQALVK
AFALEKKNGKFYFHGLEISKNEKIQVFLDSNTNNDFENQLKNTADKDIMHCIIKRNMNI
LVKVITFKENISSINIINDTNFGVQSMTGLSNRSKGQDGIYRAATTAFSFKSKELKYPE
GRYRMRFVIQSYEPFTKNLDCWVDNEEDIDVILKKSTILNLDINNDIISDISGFNSSVITY
PDAQLVPGINGKAIHLVNNESSEVIVHKAMDIEYNDMFNNFTVSFWLRVPKVSASHL
EQYGTNEYSIISSMKKHSLSIGSGWSVSLKGNNLIWTLKDSAGEVRQITFRDLPDKFN
AYLANKWVFITITNDRLSSANLYINGVLMGSAEITGLGAIREDNNITLKLDRCNNNNQ
YVSIDKFRIFCKALNPKEIEKLYTSYLSITFLRDFWGNPLRYDTEYYLIPVASSSKDVQL
KNITDYMYLTNAPSYTNGKLNIYYRRLYNGLKFIIKRYTPNNEIDSFVKSGDFIKLYVSY
NNNEHIVGYPKDGNAFNNLDRILRVGYNAPGIPLYKKMEAVKLRDLKTYSVQLKLYD
DKNASLGLVGTHNGQIGNDPNRDILIASNWYFNHLKDKILGCDWYFVPTDEGWTNDLQ

SEQ ID NO: 17

(b)

LVSKFENSVKNSNKNYFTINGLMGYYFENDFFNLNIISPTLDGNLTFSKEDINSILGNKII
KSARWIGLIKPSITGEYILSTNSPNCRVELNGEIFNLSLNTSNTVNLIQGNVYDIRIEQLM
SENQLLKNYEGIKLYWETSDIIKEIIPSEVLLKPNYSNTNEKSKFIPNNTLFSNAKLKANA
NRDTDRDGIPDEWEINGYTVMNQKAVAWDDKFAANGYKKYVSNPFKPCTANDPYT
DFEKVSGQIDPSVSMVARDPMISAYPIVGVQMERLVVSKSETITGDSTKSMSKSTSH
SSTNINTVGAEVSGSLQLAGGIFPVFSMSASANYSHTWQNTSTVDDTTGESFSQGL
SINTGESAYINPNIRYYNTGTAPVYNVTPTTTIVIDKQSVATIKGQESLIGDYLNPGGT
YPIIGEPPMALNTMDQFSSRLIPINYNQLKSIDNGGTVMLSTSQFTGNFAKYNSNGNL
VTDGNNWGPYLGTIKSTTASLTLSFSGQTTQVAVVAPNFSDPEDKTPKLTLEQALVK
AFALEKKNGKFYFHGLEISKNEKIQVFLDSNTNNDFENQLKNTADKDIMHCIIKRNMNI
LVKVITFKENISSINKNLDCWVDNEEDIDVILKKSTILNLDINNDIISDISGFNSSVITYPD
AQLVPGINGKAIHLVNNESSEVIVHKAMDIEYNDMFNNFTVSFWLRVPKVSASHLEQ
YGTNEYSIISSMKKHSLSIGSGWSVSLKGNNLIWTLKDSAGEVRQITFRDLPDKFNAY
LANKWVFITITNDRLSSANLYINGVLMGSAEITGLGAIREDNNITLKLDRCNNNNQYV
SIDKFRIFCKALNPKEIEKLYTSYLSITFLRDFWGNPLRYDTEYYLIPVASSSKDVQLKNI
TDYMYLTNAPSYTNGKLNIYYRRLYNGLKFIIKRYTPNNEIDSFVKSGDFIKLYVSYNN
NEHIVGYPKDGNAFNNLDRILRVGYNAPGIPLYKKMEAVKLRDLKTYSVQLKLYDDK
NASLGLVGTHNGQIGNDPNRDILIASNWYFNHLKDKILGCDWYFVPTDEGWTNDLQ

## Claims

1. A composition comprising a therapeutic agent linked to a polypeptide, wherein the polypeptide is a non-toxic polypeptide, for delivery of a therapeutic agent to a neuronal cell, comprising:-
a binding domain that binds to the neuronal cell and is comprised of or derived from clostridial heavy chain fragments, and
a translocation domain that translocates the therapeutic agent into the neuronal cell,
wherein the translocation domain is not a H_{N} domain of a clostridial toxin and is not a fragment or derivative of a H_{N} domain of a clostridial toxin and
wherein the therapeutic agent is for reduction of neuronal damage after ischemia/reperfusion or is for promotion of neuronal growth after damage.

2. A composition according to Claim 1, wherein the translocation domain is further a non-aggregating translocation domain as measured by size in physiological buffers.

3. A composition according to Claim 1 or 2, wherein the translocation domain is further selected from (1) a H_{N} domain of a diphtheria toxin, (2) a fragment or derivative of (1) that substantially retains the translocating activity of the H_{N} domain of a diphtheria toxin, (3) a fusogenic peptide, (4) a membrane disrupting peptide, and (5) translocating fragments and derivatives of (3) and (4).

4. A composition according to any of Claims 1 to 3, wherein the polypeptide has the binding specificity of tetanus toxin and reduced affinity to neutralising antibodies to tetanus toxin compared with the affinity to such antibodies of native tetanus toxin heavy chain.

5. A composition comprising a therapeutic agent linked to a polypeptide, wherein the polypeptide is for delivery of a therapeutic agent to a neuronal cell, comprising:-
a binding domain that binds to the neuronal cell and is comprised of or derived from clostridial heavy chain fragments, and
a translocation domain that translocates the therapeutic agent into the neuronal cell,
wherein the translocation domain is selected from (1) a H_{N} domain of a diphtheria toxin, (2) a fragment or derivative of (1) that substantially retains the translocating activity of the H_{N} domain of a diphtheria toxin, (3) a fusogenic peptide, (4) a membrane disrupting peptide, and (5) translocating fragments and derivatives of (3) and (4) and wherein the therapeutic agent is for reduction of neuronal damage after ischemia/reperfusion or is for promotion of neuronal growth after damage.

6. A composition according to Claim 5, wherein the polypeptide has reduced affinity to neutralising antibodies to tetanus toxin compared with the affinity to such antibodies of native tetanus toxin.

7. A composition comprising a therapeutic agent linked to a polypeptide, wherein the polypeptide is for delivery of a therapeutic agent to a neuronal cell, comprising:-
a binding domain that binds to the neuronal cell and is comprised of
or derived from clostridial heavy chain fragments, and
a translocation domain that translocates the therapeutic agent into the neuronal cell,
wherein the polypeptide has reduced affinity to neutralising antibodies to tetanus toxin compared with the affinity to such antibodies of native tetanus toxin and wherein the therapeutic agent is for reduction of neuronal damage after ischemia/reperfusion or is for promotion of neuronal growth after damage.

8. A composition according to any previous claim wherein the binding domain comprises a botulinum H_{C} domain.

9. A composition according to any of claims 1 to 7 wherein the binding domain comprises a tetanus H_{C} domain.

10. A composition according to any of claims 1 to 7 wherein the binding domain comprises a hybrid of a botulinum H_{C} domain and a tetanus H_{C} domain.

11. A composition according to Claim 10, based on a botulinum type B fragment (residues 859-1291) or type A fragment (residues 872-1296) or type F fragment (residues 862-1278).

12. A composition according to Claim 10, based on a botulinum type B fragment (residues 859-1291) by replacing residues 1093-1285 with the homologous sequence from tetanus toxin.

13. A composition according to Claim 10, based on a botuiinum type A fragment (residues 872-1296) by replacing residues 1103-1296 with the homologous sequence from tetanus toxin.

14. A composition according to Claim 10, based on a botulinum type F fragment (residues 862-1278) by replacing residues 1097-1273 with the homologous sequence from tetanus toxin.

15. A composition according to any of Claims 1 to 7 comprising a tetanus H_{C} domain and a diphtheria H_{N} domain.

16. A composition according to any of Claims 1 to 7 comprising a botulinum H_{C} domain and diphtheria H_{N} domain.

17. A composition according to any of Claims 1-16, wherein the therapeutic substance is chemically bound to said polypeptide.

18. A composition according to any of Claims 1-17 wherein the therapeutic substance is linked to a translocation domain of said polypeptide.

19. A composition according to any of Claims 1 to 18 wherein the therapeutic substance is an enzyme, growth factor, protein or peptide.

20. A composition according to any of Claims 18 to 19 wherein the therapeutic agent is produced as a fusion protein by recombinant technology methods.

21. A composition according to any of Claims 1 to 20 wherein the therapeutic agent is a nucleic acid useful as a gene therapeutic agent or an antisense drug.

22. A composition according to Claim 21 in which the nucleic acid is contained within a liposome or is condensed via a peptide or protein, or by condensation using a chemical coupling agent.

23. A composition according to Claim 21 in which the nucleic acid is present in the form of a recombinant virus.

24. A composition according to Claim 21 in which the virus has an altered tropism and is capable of transducing cells via a clostridial toxin receptor.

25. A method of manufacture of a composition according to any of Claims 1 to 16, comprising expressing in a host cell a nucleic acid encoding the composition.

26. Use of a composition according to any of Claims 1 to 24 for the manufacture of a medicament for the treatment of a disease state associated with neuronal cells.

## Patentansprüche

1. Zusammensetzung, die ein Heilmittel umfasst, das an ein Polypeptid gekoppelt ist, worin das Polypeptid ein nicht-toxisches Polypeptid ist, für die Übermittlung eines Heilmittels an eine neuronale Zelle, umfassend:
eine Bindungsdomäne, die an die neuronale Zelle bindet und zusammengesetzt ist
aus oder abgeleitet ist von Fragmenten der schweren Kette von Clostridien, und
eine Translokationsdomäne, die das Heilmittel in die neuronale Zelle überführt,
worin die Translokationsdomäne keine H_{N}-Domäne eines Clostridientoxins ist und kein Fragment oder Derivat einer H_{N}-Domäne eines Clostridientoxins ist und worin das Heilmittel für die Reduktion neuronaler Schädigung nach Ischämie/Reperfusion oder für die Förderung neuronalen Wachstums nach Beschädigung ist.

2. Zusammensetzung nach Anspruch 1, worin die Translokationsdomäne weiterhin eine nicht aggregierende Translokationsdomäne ist, bestimmt durch die Größe in physiologischen Puffern.

3. Zusammensetzung nach Anspruch 1 oder 2, worin die Translokationsdomäne weiterhin ausgewählt ist aus (1) einer H_{N}-Domäne eines Diphtherietoxins, (2) einem Fragment oder Derivat von (1), das im Wesentlichen die Translokationsaktivität der H_{N}-Domäne eines Diphtherietoxins behält, (3) einem fusogenen Peptid, (4) einem Peptid, das die Membran unterbricht, und (5) translozierenden Fragmenten und Derivaten von (3) und (4).

4. Zusammensetzung nach einem der Ansprüche 1 bis 3, worin das Polypeptid die Bindungsspezifität von Tetanustoxin hat und eine reduzierte Affinität für neutralisierende Antikörper für Tetanustoxin im Vergleich mit der Affinität für solche Antikörper für die schwere Kette von natürlichem Tetanustoxin hat.

5. Zusammensetzung, die ein Heilmittel umfasst, das an ein Polypeptid gekoppelt ist, worin das Polypeptid für die Übermittlung eines Heilmittels an eine neuronale Zelle ist, umfassend:
eine Bindungsdomäne, die an die neuronale Zelle bindet und zusammengesetzt ist oder abgeleitet ist von Fragmenten der schweren Kette von Clostridien, und
eine Translokationsdomäne, die das Heilmittel in die neuronale Zelle überführt,
worin die Translokationsdomäne ausgewählt ist aus (1) einer H_{N}-Domäne eines Diphtherietoxins, (2) einem Fragment oder Derivat von (1), das im Wesentlichen die Translokationsaktivität der H_{N}-Domäne eines Diphtherietoxins behält, (3) einem fusogenen Peptid, (4) einem Peptid, das die Membran unterbricht, und (5) translozierenden Fragmenten und Derivaten von (3) und (4), und worin das Heilmittel für die Reduktion von neuronaler Schädigung nach I-schämie/Reperfusion oder für die Förderung neuronalen Wachstums nach Schädigung ist.

6. Zusammensetzung nach Anspruch 5, worin das Polypeptid eine reduzierte Affinität für neutralisierende Antikörper für Tetanustoxin hat im Vergleich mit der Affinität für solche Antikörper von natürlichem Tetanustoxin.

7. Zusammensetzung, die ein Heilmittel umfasst, das an ein Polypeptid. gekoppelt ist, worin das Polypeptid für die Übermittlung eines Heilmittels an eine neuronale Zelle ist, umfassend:
eine Bindungsdomäne, die an die neuronale Zelle bindet und zusammengesetzt ist oder abgeleitet ist von Fragmenten der schweren Kette von Clostridien, und
eine Translokationsdomäne, die das Heilmittel in die neuronale Zelle überführt,
worin das Polypeptid eine reduzierte Affinität für neutralisierende Antikörper für Tetanustoxin im Vergleich mit der Affinität für solche Antikörper von natürlichem Tetanustoxin hat, und worin das Heilmittel für die Reduktion von neuronaler Schädigung nach Ischämie/Reperfusion oder für die Förderung neuronalen Wachstums nach Schädigung ist.

8. Zusammensetzung nach einem der vorangegangenen Ansprüche, worin die Bindungsdomäne eine Botulinum-H_{C}-Domäne umfasst.

9. Zusammensetzung nach einem der Ansprüche 1-7, worin die Bindungsdomäne eine Tetanus-H_{C}-Domäne umfasst.

10. Zusammensetzung nach einem der Ansprüche 1-7, worin die Bindungsdomäne ein Hybrid einer Botulinum-H_{C}-Domäne und einer Tetanus-H_{C}-Domäne umfasst.

11. Zusammensetzung nach Anspruch 10, basierend auf einem Botulinum Typ B-Fragment (Reste 859-1291) oder Typ A-Fragment (Reste 872-1296) oder Typ F-Fragment (Reste 862-1278).

12. Zusammensetzung nach Anspruch 10, basierend auf einem Botulinum Typ B-Fragment (Reste 859-1291) durch Ersetzen der Reste 1093-1285 mit der homologen Sequenz von Tetanustoxin.

13. Zusammensetzung nach Anspruch 10, basierend auf einem Botulinum Typ A-Fragment (Reste 872-1296) durch Ersetzen der Reste 1103-1296 mit der homologen Sequenz von Tetanustoxin.

14. Zusammensetzung nach Anspruch 10, basierend auf einem Botulinum Typ F-Fragment (Reste 863-1278) durch Ersetzen der Reste 1097-1273 mit der homologen Sequenz von Tetanustoxin.

15. Zusammensetzung nach einem der Ansprüche 1-7, umfassend eine Tetanus-H_{C}-Domäne und eine Diphtherie-H_{N}-Domäne.

16. Zusammensetzung nach einem der Ansprüche 1-7, umfassend eine Botulinum-H_{C}-Domäne und eine Diphtherie-H_{N}-Domäne.

17. Zusammensetzung nach einem der Ansprüche 1-16, worin die therapeutische Substanz chemisch an dieses Polypeptid gebunden ist.

18. Zusammensetzung nach einem der Ansprüche 1-17, worin die therapeutische Substanz an eine Translokationsdomäne dieses Polypeptids gekoppelt ist.

19. Zusammensetzung nach einem der Ansprüche 1-18, woin die therapeutische Substanz ein Enzym, Wachstumsfaktor, Protein oder Peptid ist.

20. Zusammensetzung nach einem der Ansprüche 18-19, worin das Heilmittel hergestellt ist als ein Fusionsprotein durch Verfahren der Rekombinationstechnik.

21. Zusammensetzung nach einem der Ansprüche 1-20, worin das Heilmittel eine Nukleinsäure ist, die nützlich ist als ein Genheilmittel oder ein antisense-Arzneimittel.

22. Zusammensetzung nach Anspruch 21, bei der die Nukleinsäure in einem Liposom enthalten ist oder kondensiert ist über ein Peptid oder Protein oder kondensiert ist durch Kondensation unter Verwendung eines chemischen Kupplungsmittels.

23. Zusammensetzung nach Anspruch 21, bei der die Nukleinsäure in der Form eines rekombinanten Virus vorhanden ist.

24. Zusammensetzung nach Anspruch 21, bei der das Virus einen veränderten Tropismus hat und fähig ist, Zellen zu transduzieren über einen Clostridientoxinrezeptor.

25. Verfahren zur Herstellung einer Zusammensetzung nach einem der Ansprüche 1-16, umfassend die Expression einer Nukleinsäure, die die Zusammensetzung kodiert, in einer Wirtszelle.

26. Verwendung einer Zusammensetzung nach einem der Ansprüche 1-24 für die Herstellung eines Medikamentes für die Behandlung eines Krankheitszustandes im Zusammenhang mit neuronalen Zellen.

## Revendications

1. Composition comprenant un agent thérapeutique lie à un polypeptide, dans laquelle le polypeptide est un polypeptide non-toxique, destiné à apporter un agent thérapeutique à une cellule neuronale, comprenant :
un domaine de liaison, qui se lie à la cellule neuronale et est composé ou derive de fragments de chaînes lourdes clostridiennes et
un domaine de translocation, qui transloque l'agent therapeutique dans la cellule neuronale,
dans laquelle le domaine de translocation n'est pas un domaine H_{N} d'une toxine clostridienne et n'est pas un fragment ou un dérivé d'un domaine H_{N} d'une toxine clostridienne, et dans laquelle l'agent therapeutique est destiné à la réduction de l'endommagement neuronal apres une ischemie / réperfusion ou est destiné à la promotion de la croissance neuronale après endommagement.

2. Composition selon la revendication 1, dans laquelle le domaine de translocation est en outre un domaine de translocation non-agrégeant, tel que mesure par la taille dans des tampons physiologiques.

3. Composition selon la revendication 1 ou 2, dans laquelle le domaine de translocation est en outre choisi parmi (1) un domaine H_{N} d'une toxine de diphtérie, (2) un fragment ou un dérivé de (1), qui retient sensiblement l'activité de translocation du domaine H_{N} d'une toxine de diphtérie, (3) un peptide fusogénique, (4) un peptide perturbant les membranes, et (5) des fragments transloquants et des dérivés de (3) et (4).

4. Composition selon l'une quelconque des revendications 1 à 3, dans laquelle le polypeptide presente la spécificité de liaison de la toxine du tétanos et une affinité réduite pour les anticorps neutralisants dirigés contre une anti-toxine du tetanos comparée à l'affinité d'une chaîne lourde de toxine du tétanos pour de tels anticorps.

5. Composition comprenant un agent thérapeutique lié à un polypeptide, dans laquelle le polypeptide est destiné à apporter un agent thérapeutique à une cellule neuronale, comprenant :
un domaine de liaison, qui se lie à la cellule neuronale et est composé ou dérivé de fragments de chaînes lourdes clostridiennes et
un domaine de translocation, qui transloque l'agent thérapeutique dans la cellule neuronale,
dans laquelle le domaine de translocation est choisi parmi (1) un domaine H_{N} d'une toxine de diphtérie, (2) un fragment ou un dérivé de (1), qui retient sensiblement l'activité de translocation du domaine H_{N} d'une toxine de diphtérie, (3) un peptide fusogénique, (4) un peptide perturbant les membranes, et (5) des fragments transloquants et des dérivés de (3) et (4), et dans laquelle l'agent thérapeutique est destiné à la réduction de l'endommagement neuronal après une ischémie / réperfusion ou est destiné à la promotion de la croissance neuronale après endommagement.

6. Composition selon la revendication 5, dans laquelle le polypeptide présente une affinité réduite pour les anticorps neutralisants anti-toxine du tétanos, comparée à l'affinité de la toxine du tétanos native pour de tels anticorps.

7. Composition comprenant un agent thérapeutique lié à un polypeptide, dans laquelle le polypeptide est destiné à apporter un agent thérapeutique à une cellule neuronale, comprenant :
un domaine de liaison, qui se lie à la cellule neuronale et est composé ou dérivé de fragments de chaînes lourdes clostridiennes et
un domaine de translocation, qui transloque l'agent thérapeutique dans la cellule neuronale,
dans laquelle le polypeptide présente une affinité réduite pour les anticorps neutralisants anti-toxine du tétanos comparée à l'affinité de la toxine du tétanos native pour de tels anticorps, et dans laquelle l'agent thérapeutique est destiné à la réduction de l'endommagement neuronal après une ischémie/réperfusion ou est destiné à la promotion de la croissance neuronale après endommagement.

8. Composition selon l'une quelconque des revendications précédentes, dans laquelle le domaine de liaison comprend un domaine H_{C} botulinique.

9. Composition selon l'une quelconque des revendications 1 à 7, dans laquelle le domaine de liaison comprend un domaine H_{C} tétanique.

10. Composition selon l'une quelconque des revendications 1 à 7, dans laquelle le domaine de liaison comprend un hybride d'un domaine H_{C} botulinique et d'un domaine H_{C} de tétanos.

11. Composition selon la revendication 10, basée sur un fragment botulinique de type B (résidus 859-1291) ou un fragment de type A (résidus 872-1296) ou un fragment de type F (résidus 862-1278).

12. Composition selon la revendication 10, basée sur un fragment botulinique de type B (résidus 859-1291) en remplaçant les résidus 1093-1285 avec la séquence homologue issue de la toxine du tétanos.

13. Composition selon la revendication 10, basée sur un fragment botulinique de type A (résidus 872-1296) en remplaçant les résidus 1103-1296 avec la séquence homologue issue de la toxine du tétanos.

14. Composition selon la revendication 10, basée sur un fragment botulinique de type F (résidus 862-1278) en remplaçant les résidus 1097-1273 avec la séquence homologue issue de la toxine du tétanos.

15. Composition selon l'une quelconque des revendications 1 à 7 comprenant un domaine H_{C} tétanique et un domaine H_{N} diphtérique.

16. Composition selon l'une quelconque des revendications 1 à 7 comprenant un domaine H_{C} botulinique et un domaine H_{N} diphtérique.

17. Composition selon l'une quelconque des revendications 1 à 16, dans laquelle la substance thérapeutique est liée chimiquement audit polypeptide.

18. Composition selon l'une quelconque des revendications 1 à 17, dans laquelle la substance thérapeutique est liée à un domaine de translocation dudit polypeptide.

19. Composition selon l'une quelconque des revendications 1 à 18, dans laquelle la substance thérapeutique est une enzyme, un facteur de croissance, une protéine ou un peptide.

20. Composition selon l'une quelconque des revendications 18 à 19, dans laquelle l'agent thérapeutique est produit sous forme de protéine de fusion par des procédés de technologie recombinante.

21. Composition selon l'une quelconque des revendications 1 à 20, dans laquelle l'agent thérapeutique est un acide nucléique utile en tant qu'agent thérapeutique de gène ou de drogue antisens.

22. Composition selon la revendication 21, dans laquelle l'acide nucléique est contenu à l'intérieur d'un liposome ou est condensé via un peptide ou une protéine, ou par condensation en utilisant un agent de couplage chimique.

23. Composition selon la revendication 21, dans laquelle l'acide nucléique est présent sous la forme d'un virus recombinant.

24. Composition selon la revendication 21, dans laquelle le virus présente un tropisme modifié est capable de transducter des cellules via un récepteur de toxine clostridienne.

25. Procédé de fabrication d'une composition selon l'une quelconque des revendications 1 à 16, comprenant l'expression dans une cellule hôte d'un acide nucléique codant la composition.

26. Utilisation d'une composition selon l'une quelconque des revendications 1 à 24 pour la fabrication d'un médicament destiné au traitement d'un état de maladie associé aux cellules neuronales.
